(19) Europäisches Patentamt
European Patent Office
Office européen des brevets

(11) Publication number: **0 049 618**
**B1**

(12) # EUROPEAN PATENT SPECIFICATION

(45) Date of publication of patent specification: **14.08.85**

(51) Int. Cl.[4]: **C 07 D 277/28,** A 61 K 31/425 // C07D277/56, C07D417/06

(21) Application number: **81304572.1**

(22) Date of filing: **02.10.81**

(54) **Improvements in or relating to 2,4-disubstituted thiazole derivatives.**

(30) Priority: **02.10.80 US 193192**

(43) Date of publication of application:
**14.04.82 Bulletin 82/15**

(45) Publication of the grant of the patent:
**14.08.85 Bulletin 85/33**

(84) Designated Contracting States:
**AT BE CH DE FR GB IT LI LU NL SE**

(56) References cited:
**EP-A-0 009 061**
**FR-A-2 128 505**
**FR-A-2 229 417**
**FR-A-2 343 730**
**US-A-4 151 288**
**US-A-4 200 578**

**The file contains technical information submitted after the application was filed and not included in this specification**

(73) Proprietor: **ELI LILLY AND COMPANY**
**307, East McCarty Street**
**Indianapolis Indiana 46285 (US)**

(72) Inventor: **Pioch, Richard Paul**
**3750, Briarwood Drive**
**Indianapolis Indiana 46240 (US)**

(74) Representative: **Crowther, Terence Roger et al**
**Erl Wood Manor**
**Windlesham Surrey GU20 6PH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 049 618 B1

**Description**

This invention relates to certain novel thiazole derivatives which have been found to be effective histamine $H_2$ receptor antagonists, and which are therefore useful in the treatment of peptic ulcers.

The most successful $H_2$ antagonist hitherto developed in the imidazole derivative cimetidine: N-cyano-N′-methyl-N″-[2-([(5-methyl-1H-imidazol-4-yl)methyl]thio)ethyl]guanidine, which may be represented by the structural formula:

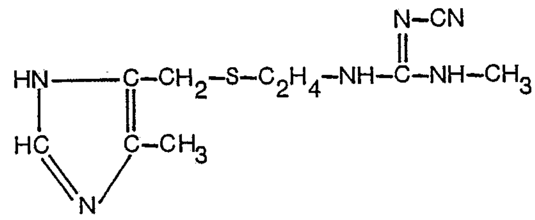

and see, for instance, *Brit. J. Pharmacol. 53*, 435p (1975). This compound has achieved great success in the treatment of peptic ulcers in humans.

US—A—4200578 discloses certain N-alkynyl-N′-{*w*-[(optionally substituted thiazolyl)methylthio]alkyl}-derivatives of N″-cyanoguanidine and FR—A—2128505 discloses N-substituted-N′-{*w*-[(optionally substituted thiazol-2-yl)methylthio]alkyl}-derivatives of thiourea.

In accordance with the invention, it has now been discovered that thiazole derivatives of formula (I):

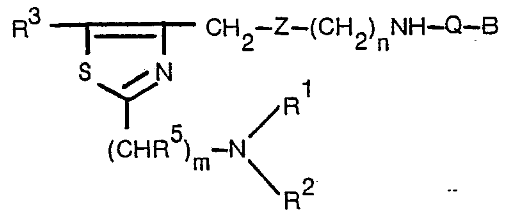

(I)

wherein each of $R^1$ and $R^2$ independently represent hydrogen, $C_{1-4}$ alkyl, benzyl or benzoyl; or taken together with the adjacent nitrogen atom, form a saturated heterocyclic ring containing from 5 to 7 ring atoms;

$R^3$ is hydrogen or $C_{1-4}$ alkyl;

Z is O, S, or $CH_2$;

n is 2 or 3 when Z is O or S and n is 1, 2 or 3 when Z is $CH_2$;

$R^5$ is hydrogen or $C_{1-4}$ alkyl;

m is 1, 2 or 3;

```
      A    O=C—C=O
      ‖      |  |
Q is C or    C=C    ;
```

wherein A is N—CN, N—$NO_2$, CH—$NO_2$, S, O, NH, N—$SO_2$-aryl, N—$SO_2$—$C_{1-4}$ alkyl, N—CO—$NH_2$, N—CO—$C_{1-4}$ alkyl, N—$CO_2$—$C_{1-4}$ alkyl; CH—$SO_2$-aryl or CH—$SO_2$—$C_{1-4}$ alkyl, wherein aryl is phenyl, halophenyl, $C_{1-4}$ alkylphenyl or $C_{1-4}$ alkoxyphenyl; and

B is $NRR^6$, wherein R and $R^6$ are independently hydrogen, $C_{1-5}$ alkyl, $C_{3-6}$ cycloalkylmethyl, hydroxy $C_{2-5}$ alkyl, $C_{3-6}$ cycloalkyl, alkoxyalkyl or dialkylaminoalkyl wherein the total number of carbons is less than 8 and there is at least a two carbon chain between the hetero atoms; or $YR^4$, wherein Y is oxygen or sulfur and $R^4$ is $C_{1-5}$ alkyl, —$CH_2$ $C_{2-4}$ alkenyl or benzyl; and pharmaceutically-acceptable salts of compounds in which B is $NRR^6$, are particularly effective $H_2$ receptor antagonists, or are useful intermediates in the preparation of such compounds.

Preferred compounds are those wherein $R^1$ and $R^2$ individually represent hydrogen or $C_{1-3}$ alkyl, one only of $R^1$ and $R^2$ may represent benzyl or benzoyl, or $R^1$ and $R^2$ taken together with the adjacent nitrogen atom represent a piperidino, pyrrolidino or morpholino group, provided that only one of $R^1$ and $R^2$ can be hydrogen when Z is $CH_2$; $R^3$ is hydrogen or $C_{1-3}$ alkyl; $R^4$ is $C_{1-5}$ alkyl, $R^5$ is hydrogen or methyl; A is N—CN, N—$NO_2$, CH—$NO_2$, S, O, NH, N—$SO_2$-aryl, N—$SO_2$—$C_{1-3}$ alkyl, N—CO—$NH_2$, N—CO—$C_{1-3}$ alkyl, N—$CO_2$—$C_{1-3}$ alkyl, CH—$SO_2$-aryl or CH—$SO_2$—$CH_3$, where aryl is phenyl, halophenyl, $C_{1-3}$ alkylphenyl or $C_{1-3}$ alkoxyphenyl, $R^6$ is hydrogen; provided that when B is $YR^4$, Q is C=A; and the pharmaceutically-acceptable, non-toxic acid-addition salts thereof.

Further preferred features possessed by the compounds of the invention are those listed below:

(a) Z is S;

(b) n is 2;

(c) $R^3$ is hydrogen;

(d) $R^5$ is hydrogen;
(e) m is 1;
(f) $R^1$ and $R^2$ are methyl;

$R_1$
/
(g) the —$(CHR^5)_m$—N group is dimethylaminomethyl;
\
$R_3$

(h) A is NCN or $CHNO_2$;
(i) B is $NRR^6$ where R is hydrogen and $R^6$ is $C_{1-4}$ alkyl; and
(j) B is methylamino.

The presently preferred compounds of the invention are:

N-methyl-N′-2-([2-(dimethylaminomethyl)-4-thiazolyl]methylthio)ethyl-N″-cyanoguanidine

N-methyl-N′-([2-(dimethylaminomethyl)-4-thiazolyl]methylthio)ethyl-2-nitro-1,1-ethenediamine, and their pharmaceutically-acceptable salts.

Compounds of formula (I) in which B is $NRR^6$ are pharmacologically-active compounds in which B is $YR^4$ are useful intermediates.

Other bases of formula (I) include:

N-ethyl-N′-[2-(2-dimethylaminoethyl)-5-methyl-4-thiazolyl)methylthio]ethylguanidine

N-cyclopropylmethyl-N′-(3-(3-([(2-(methylethylaminomethyl)-4-thiazolyl)]methylthio)propylguanidine.

N-cyclohexyl-N′-[2-(2-aminomethyl-5-n-propyl-4-(thiazolyl)methylthio]ethyl-N″-nitroguanidine

N-cyclobutylmethyl-N′-[(2-(diethylaminoethyl)-4-thiazolyl]methylthio)ethyl-N″-p-chlorophenylsulfonylguanidine

N-n-propyl-N′-2-([2-(methylaminomethyl)-5-methyl-4-thiazolyl]methylthio)ethylthiourea

N-isopropyl-N′-3-([2-(ethylaminoethyl)-5-ethyl-4-thiazolyl]methylthio)propylguanidine

N-ethyl-N′-2-([2-(diethylaminopropyl)-5-methyl-4-thiazolyl]methylthio)ethyl 2-(o-bromophenylsulfonyl)-1,1-ethenediamine or [1-(o-bromophenylsulfonyl)-2-ethylamino-2-(2-([2-(diethylaminopropyl)-5-ethyl-4-thiazolyl]methylthio)ethylamino)ethylene

N-cyclopentylmethyl-N′-2-([2-(isopropylaminomethyl)-4-thiazolyl)]methylthio)ethyl 2-methanesulfonyl-1,1-ethenediamine

N-pentyl-N′-3-([2-diethylaminoethyl)-5-propyl-4-thiazolyl]methylthio)propyl 2-nitro-1,1-ethenediamine.

N-(3-methylbutyl)-N′-2-([2-(n-propylaminomethyl)-5-ethyl-4-thiazolyl]methylthio)ethyl 2-o-tolylsulfonyl-1,1-ethenediamine

N-isobutyl-N′-2-([2-(ethyl-n-propylaminomethyl)-5-n-propyl-4-thiazolyl)methylthio)ethyl 2-nitro-1,1-ethenediamine,

N-n-propyl-N′-2-[(2-piperidinomethyl-4-thiazolyl)methylthio]ethyl-N″-cyanoguanidine

N-methoxyethyl-N′-3-([2-(aminomethyl)-4-thiazolyl]methylthio)propyl 2-nitro-1,1-ethenediamine

N-(3-hydroxypropyl)-N′-4-[2-(ethylaminomethyl)-5-methyl-4-thiazolyl]-1-butyl-N″-cyanoguanidine

N-cyclobutylmethyl-N′-5-[(2-(dimethylaminomethyl)-4-thiazolyl]-1-pentyl 1,2-diamino-3,4-dioxo-1-cyclobutene or 1-(5-(2-(dimethylaminomethyl)-4-thiazolyl)-1-pentylamino)-2-cyclobutylmethylamino-3,4-dioxo-1-cyclobutene

N-cyclopentyl-N′-2-[(4-morpholinomethyl-5-ethyl-4-thiazolyl)methyloxy]ethylguanidine

N-cyclohexyl-N′-3-([2-(1-pyrrolidinomethyl)-4-thiazolyl]methyloxy)propylurea.

N-cyclopropylmethyl-N′-3-([2-(methylaminopropyl)-4-thiazolyl]methyloxy)propylthiourea

N-dimethylaminoethyl-N′-3-[2-(ethylaminoethyl)-4-thiazolyl]propylguanidine.

N-methyl-N′-3-([2-(diethylaminomethyl)-5-methyl-4-thiazolyl]methyloxy)propyl-N″-nitroguanidine.

N-isopropyl-N′-2-([2-(di-n-propylaminomethyl)-4-thiazolyl]methylthio]ethyl-N″-methoxycarbonylguanidine

N-2-methylbutyl-N′-5-[2-(diethylaminoethyl)-4-thiazolyl]-1-pentyl-N″-acetylguanidine

N-n-butyl-N′-4-[2-(1-pyrrolidino)methyl)-4-thiazolyl]butyl-N″-aminocarbonylguanidine

N-methyl-N′-2-([2-(4-morpholinomethyl)-5-methyl-4-thiazolyl]methylthio)ethyl 2-nitro-1,1-ethenediamine

N-ethyl-N′-2-([2-(1-pyrrolidinomethyl)-4-thiazolyl]methylthio)ethyl 2-methanesulfonyl-1,1-ethenediamine.

In Formula (I), the term $C_{1-4}$ alkyl includes methyl, ethyl, n-propyl, isopropyl and n-butyl. Thus, the term $C_{1-4}$ alkylphenyl would include 0, m and p-tolyl, o, m and p-ethylphenyl. Similarly, the term $C_{1-4}$ alkoxyphenyl includes o, m, p-anisyl, o, m, and p-ethoxyphenyl. The term halophenyl includes o, m and p-chlorophenyl, bromophenyl, fluorophenyl and iodophenyl.

The term $C_{1-5}$ alkyl includes all of the above $C_{1-4}$ alkyl radicals, as well as n-amyl, isoamyl, 2-methylbutyl and 2-methyl-2-butyl radicals. The term $C_{3-6}$ cycloalkyl includes cyclobutyl, cyclopropyl, cyclopentyl, cyclohexyl and methylcyclopentyl radicals.

Illustrative of the alkoxyalkyl or dialkylaminoalkyl groups having less than a total of eight carbons, and

the heteroatom of which is separated from the nitrogen atom to which the group is attached by at least a two carbon chain are 2-methoxyethyl, isopropoxyethyl, 3-ethoxy-2-methylpropyl, 2-(2-pentyloxyethyl) 2-dimethylaminoethyl, diethylaminoethyl and 2-methylpropylamino-2-propyl.

The pharmaceutically-acceptable salts of the compounds of this invention include salts derived from inorganic acids such as hydrochloric acid, phosphoric acid, sulfuric acid, hydrobromic acid, hydroiodic acid, phosphorous acid, as well as salts derived from organic acids such as aliphatic mono and dicarboxylic acids, phenyl-substituted alkanoic acids, hydroxy alkanoic and alkandioic acids, aromatic acids, aliphatic and aromatic sulfonic acids. Such pharmaceutically-acceptable salts thus include sulfate, pyrosulfate, bisulfate, sulfite, bisulfite, nitrate, phosphate, monohydrogenphosphate, dihydrogenphosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, mandelate, butyne-1,4-dioate, hexyne-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate lactate, β-hydroxybutyrate, glycollate, malate, tartrate, methanesulfonate, propanesulfonate, naphthalene-1-sulfonate and naphthalene-2-sulfonate.

Compounds of formula (I) have at least one basic center, the aminoalkyl group at C-2 of the thiazole ring but may have a second or third basic salt-forming group. For example, the substituted amidine terminal group can also have nitrogens present which are, depending on the substitution pattern, sufficiently basic to form salts with nontoxic acids.

The compounds of this invention wherein Z is S or O are conveniently prepared from a 2-[(2-aminoalkyl-4-thiazolyl)methoxy or methylthio]alkyl amine. The preparation of these starting materials is illustrated in Flow Chart A below using a compound in which the heteroatom is sulfur for exemplary purposes only.

FLOW CHART A

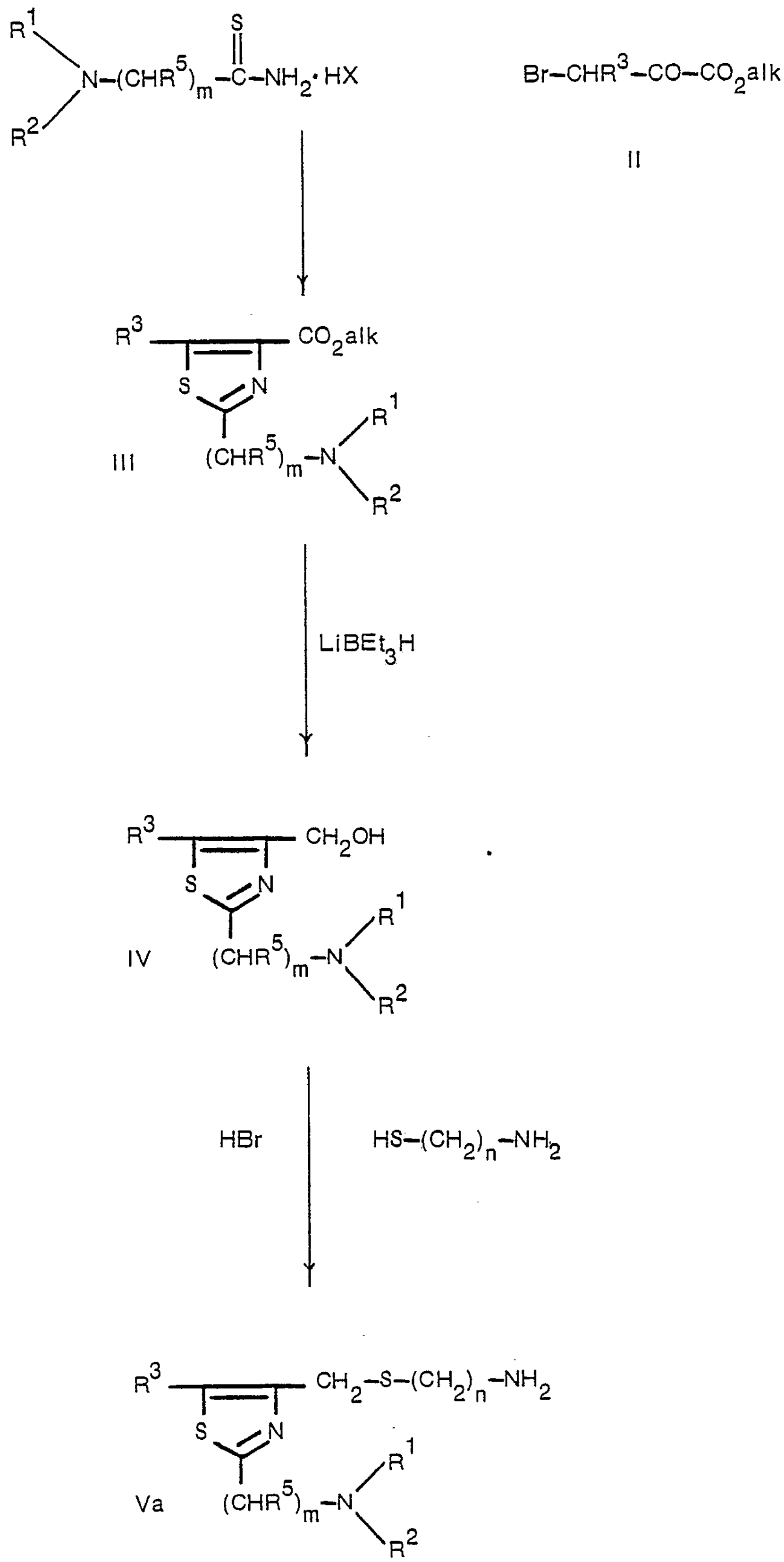

In the above Flow Chart, alk is conveniently methyl or ethyl and $R^1$, $R^2$, $R^3$, $R^5$, m and n have the same meaning as hereinabove.

In accordance with the above procedure, an acid addition salt of an aminoalkylthioacetamide is reacted with a beta-bromo-alpha-ketoester (II) such as ethyl bromopyruvate ($R^3$=H) to yield an alkyl (methyl or ethyl) 2-(aminoalkyl)-4-thiazolecarboxylate (III). Reduction of this ester with a suitable hydride reducing agent such as lithium triethylborohydride, lithium aluminum hydride, sodium borohydride or diisobutylaluminumhydride yields the corresponding hydroxymethyl compound (IV). Reaction of the 4-hydroxymethylthiazole with cysteamine or its higher homologue ω-thiopropylamine in the presence of acid yields directly a 2-[(2-aminoalkyl-4-thiazolyl)methylthio]alkylamine (Va) optionally substituted with an alkyl group in the 5-position of the thiazole ring.

In the process indicated in Flow Chart A, in going from IV to V, the hydroxymethyl group can be reacted with, for example, thionylchloride to yield a 4-chloromethylthiazole and this compound reacted with the sodium salt of the particular mercaptoalkylamine. In fact, any standard leaving group (a group labile to nucleophilic displacement) can be employed here in place of chloro in the chloromethyl side chain including for example p-tosyloxy, mesyloxy (methanesulfonyloxy), bromo or iodo.

Alternatively, the 4-chloromethylthiazole hydrochloride (or other suitable acid addition salt) can be fused with a mercaptoalkylamine salt such as a hydrochloride salt to yield the desired primary amine Va—Z=S).

If it is desired to prepare the side chain oxygen analogue of Va (Z=O), a process utilizing 2-chloroethylamine or 3-chloropropylamine to react with the 4-thiazolemethanol, under basic conditions, can be employed as well as can the analogous Williamson ether process using the sodium salt of the hydroxyalkylamine with a 4-thiazolylmethyl halide.

In flow chart A in the substituted aminothioacetamide hydrohalide of the structure

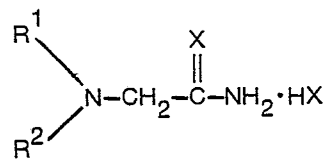

when $R^1$ and $R^2$ are $C_{1-4}$ alkyl, the compounds are known as, for example, dimethylaminothioacetamide, diethylaminothioacetamide, etc. and can be prepared by the method described in *J. Org. Chem.,* (Russia), *6,* 884 (1970) in English.

Several pathways are available for preparing the compounds of this invention. These pathways or synthetic routes may utilize an amine of the generalized formula:

V 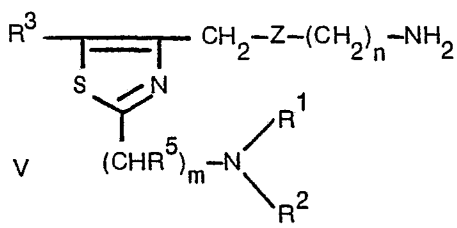

wherein Z is S, O or $CH_2$, as a starting material.

These routes are illustrated in Flow Charts B, C and D below. According to Flow Chart B, a starting primary amine, the ultimate product of Flow Chart A (Va—Z=S), is reacted with, for example, an N-alkyl, (cycloalkyl, cycloalkyl-substituted-alkyl, alkoxyalkyl or dialkylaminoalkyl) 1-methylthio-2-nitroetheneamine. During the reaction, the elements of methylmercaptan are lost and the final desired product (VIIa) is an N-2-[(2-aminoalkyl-5-optionally-substituted-4-thiazolyl)methylthio]alkyl-N'-alkyl (cycloalkyl, cycloalkylalkyl, alkoxyalkyl or dialkylaminoalkyl) 2-nitro-1,1-diaminoethylene (or ethenediamine). Similarly, the primary amine (Va) can be reacted with an S-methyl-N-alkyl (cycloalkyl, cycloalkylalkyl, alkoxyalkyl or dialkylaminoalkyl)-N'-cyanoisothiourea to form the desired product (VIa) — an N-alkyl, (cycloalkyl, cycloalkylalkyl, alkoxyalkyl or dialkylaminoalkyl)-N'-2-[(2-aminoalkyl-5-optionally substituted-4-thiazolyl)methylthio]ethyl-N''-cyanoguanidine.

## FLOW CHART B

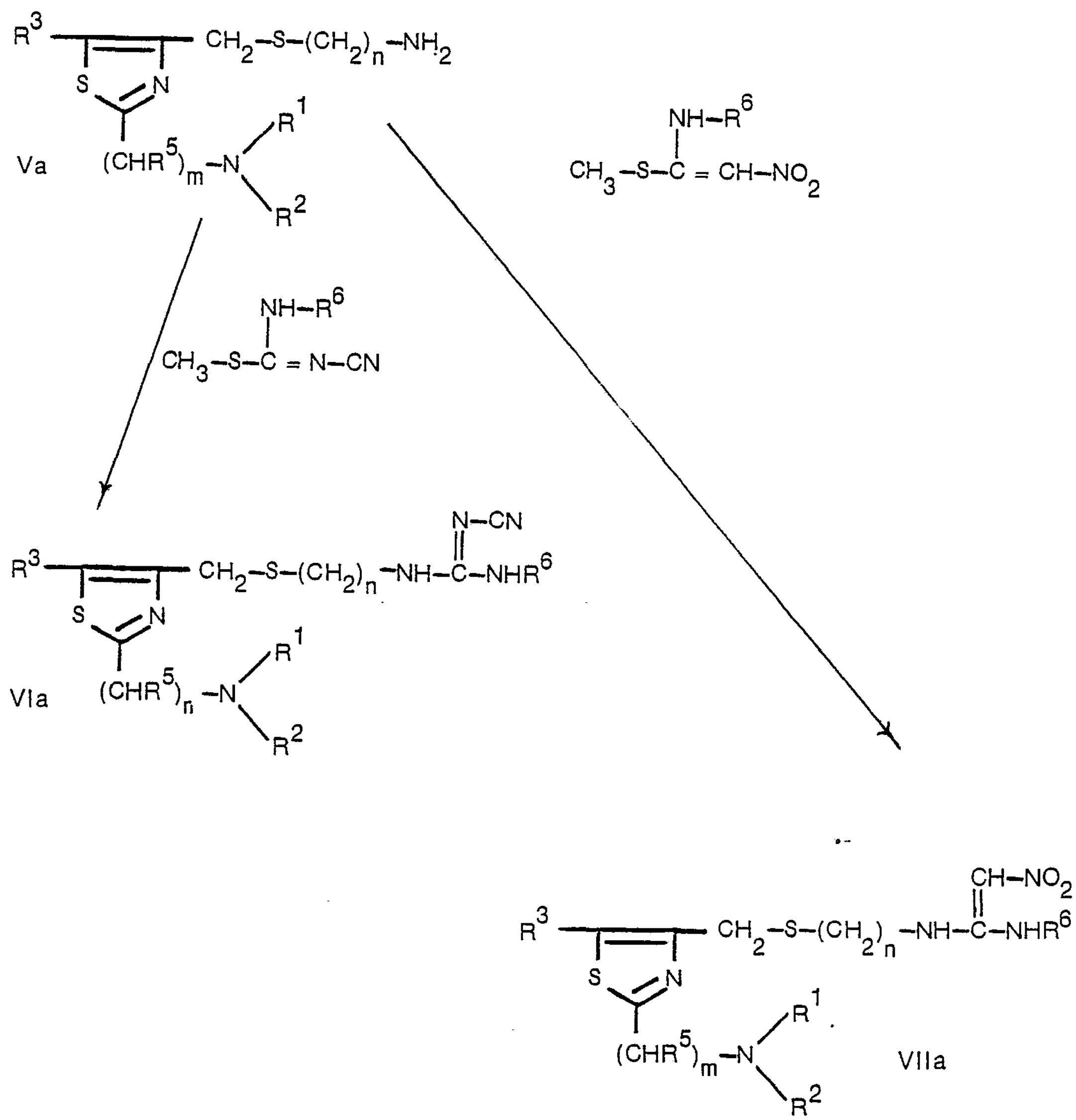

Va

VIa

VIIa

wherein $R^1$—$R^6$, m and n have the same meaning as hereinabove.

Those skilled in the art will appreciate that in the above reaction sequence, and indeed in other synthetic procedures described hereinafter, at least one of $R^1$and $R^2$ should be other than hydrogen to reduce the incidence of undesirable side-reactions.

In the above reactions, it is apparent that in place of an N-alkyl etc.-1-methylthio-2-nitroethyleneamine, an N-alkyl etc. 1-methylthio-2-methylsulfonylethyleneamine (or 2-phenylsulfonylethyleneamine) could be used. If it is desired to prepare a compound of structure (I) wherein

Q is 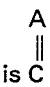

and A is N—$SO_2$-phenyl, the reagent used to prepare such N''-phenylsulfonylguanidines is dimethyl N-phenylsulfonylimidodithiocarbonate prepared by the general procedure of *Ber., 99*, 2885 (1966). The methylsulfonylguanidines are produced from a dialkyl N-methylsulfonylimidodithiocarbonate prepared in the same fashion. Similarly, it is apparent that an N-alkyl, (cycloalkyl, cycloalkylalkyl, hydroxyalkyl, alkoxyalkyl or dialkylaminoalkyl) 1-methylthio-2-arylsulfonylethyleneamine (or 2-methylsulfonylethyleneamine) could be used in place of the N-alkyl etc.-1-methylthio-2-nitroethyleneamine of the above flow chart. 2-Arylsulfonyl-1-methylthioethyleneamine, the intermediate containing a sulfonyl group, can be prepared by

reacting, for example, a 2-arylsulfonyl-1,1-bis-methylthioethylene (prepared by the method of *Bull., Soc. Chem. FR., 637*, (1973)) with one mole of an amine $NH_2R^6$. The 2-methylsulfonyl derivatives useful as intermediates can be prepared in the same fashion.

Obviously, compounds corresponding to VI and VII in which O replaces S in the bridging group are prepared by substituting a 2-aminoalkyl-4-thiazolylmethoxyalkylamine for Va in Flow Chart B.

Thus, in the general case, compounds of formula (I) in which B is $NRR^6$ may be prepared by reacting the amine intermediate of formula V with a compound of formula $L^1QB$ where $L^1$ is a leaving group, preferably for ease of preparation, a $C_{1-5}$ alkylthio, benzylthio or $C_{2-4}$ alkenylmethylthio group; Q and B being as previously defined.

The reaction should be effected in a polar solvent such as water, a $C_{1-4}$ alkanol or acetonitrile, preferably at a temperature of from 20 to 100°C, most preferably 40 to 50°C.

An example of an alternate method of preparation of the compounds of this invention is illustrated in Flow Chart C. According to this procedure, the same requisite thiazole intermediate (V) is reacted with a 1,1-bis-methylthio-2-nitro (or arylsulfonyl or methylsulfonyl) ethylene to produce an N-2-[(2-aminoalkyl-5-optionally-substituted-4-thiazolyl)methylthio]ethyl 1-amino-1-methylthio-2-nitro (or arylsulfonyl or methylsulfonyl)ethyleneamine.

FLOW CHART C

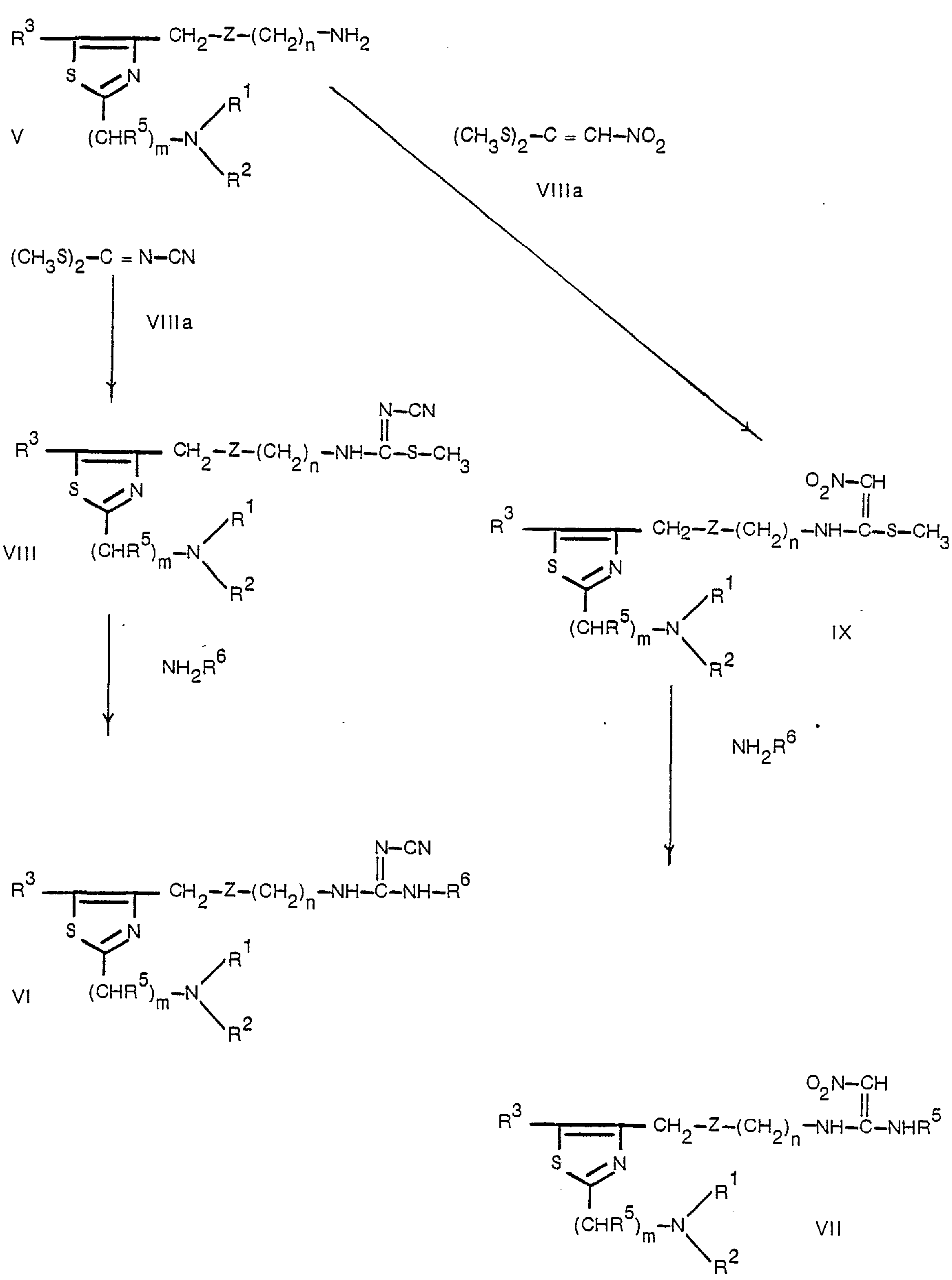


V VIII IX VI VII

wherein Z, $R^1$—$R^6$, m and n have the same meaning as hereinabove.

According to Flow Chart C, reaction of the methylmercapto compound VIII or IX with a primary amine $NH_2R^6$ yields the desired product. For example, dimethyl cyanodithioimidocarbonate will react with the

thiazolylmethylthioalkylamine or other thiazolyl side chain amine V to produce an N-2-[(2-aminoalkyl-5-optionally-substituted-4-thiazolyl)methylthio]ethyl (or propyl)-S-methyl-N'-cyanopseudothiourea (VIII where Z is S). Reaction of this compound with the primary amine $NH_2R^6$ again yields the desired product VI. Compounds in which A is CH—$NO_2$ etc. as in IX are prepared similarly and react similarly to yield an analogous final product having an ethenediamine terminal group as in VII.

In going from V to IX, a 1-methylsulfinyl-1-methylmercapto-2-nitroethylene can be used in place of 1,1-bis-methylmercapto-2-nitroethylene to yield the same intermediate IX since a methylsulfinyl group is displaced preferentially to a methylmercapto group.

Following the above procedure, in certain instances, a reactant such as VIIIa can be employed in which an $OCH_3$ group replaces the $SCH_3$. This methoxy group is replaceable by the amine $NH_2R^6$ as is the S—$CH_3$ group illustrated above. For example, a compound of the formula $(CH_3O)_2$—C=A wherein A is N—CN can be employed. Similarly, other intermediates of formula (I) in which B represents additional $YR^4$ groups other than methylthio can be prepared by using appropriate reagents of the type: $(R^4Y)_2$—C=A.

As was the case with Flow Chart B, the procedures of Flow Chart C can be modified to produce compounds according to (I) above in which Z is O by utilizing an appropriate starting material containing oxygen in the side chain.

Thus, in the general case, a compound of formula:

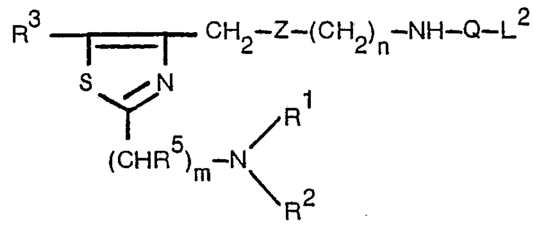

where $L^2$ is a leaving group, preferably a group of formula $YR^4$, can be reacted with an amine of formula $HNRR^6$ to yield compounds of formula (I).

The reaction is preferably effected at a temperature from 20 to 100°C in a polar solvent such as water on a $C_{1-4}$ alkanol.

A third type of formula (I) compound coming within the scope of the above formula are thioureas wherein A is S. An example of the preparation of these compounds is illustrated in Flow Chart D.

FLOW CHART D

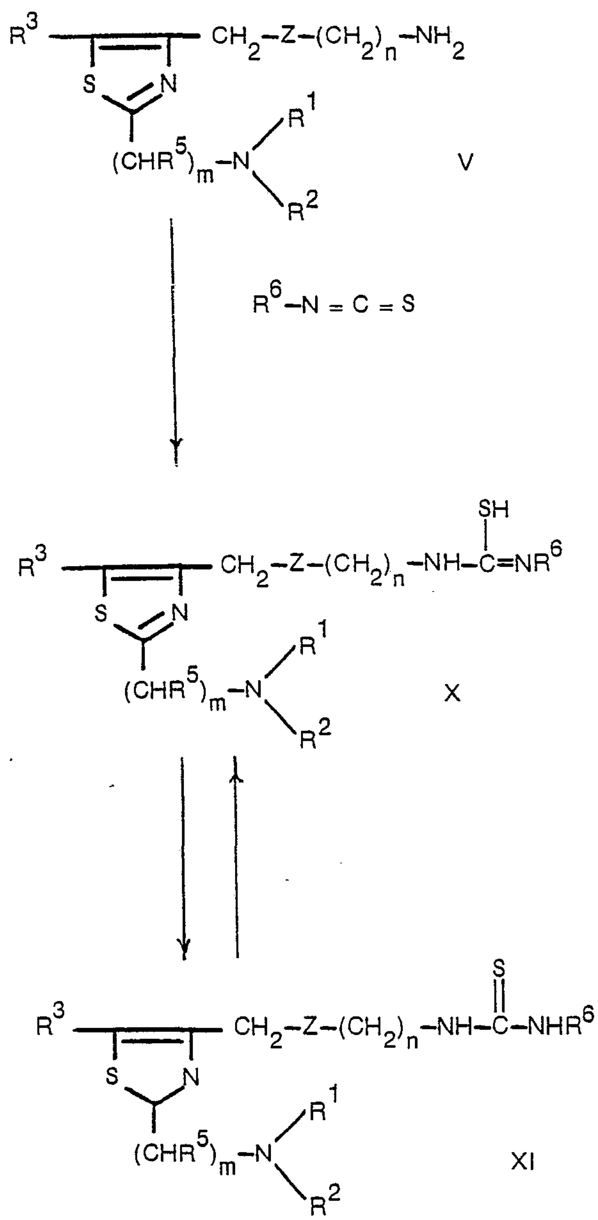


V

X

XI

wherein $R^6$, $R^1$, $R^2$, $R^3$, $R^5$, Z, n and m have their previously assigned meanings.

According to Flow Chart D, the starting amine, (V), for example a thiazolylmethylthioalkylamine (Z=S), is reacted with a suitably substituted isothiocyanate to yield directly the isothiourea (X) which compound is in equilibrium with the thiourea itself (XI). Similarly, an isocyanate, $R^6$—N=C=O, can be used to prepare the corresponding urea.

In the general case, compounds of formula (I) in which Q is C=A, and $R^6$ is hydrogen, may be prepared by reacting a compound of formula (V) with a reagent of formula

$$R^6\text{—N=C=A}$$

The reaction is preferably effected in a polar solvent such as water, a $C_{1-4}$ alkanol or acetonitrile. When A is oxygen, the reaction should normally be effected in acetonitrile. Preferred reaction temperatures range from 20 to 100°C, most preferably from 40 to 50°C.

Compounds according to (I) above in which Q is a 3,4-dioxo-1,2-cyclobutenediyl radical can be prepared in a fashion more or less analogous to the preparation of the corresponding ethenediamines of Flow Chart C, in that the bivalent molecule, 1,2-dimethoxy-3,4-dioxocyclobutene, can be reacted with a 2-aminoalkyl-4-thiazolylmethylthio(or oxy)alkyl amine to yield a 1-[2-aminoalkyl-4-thiazolylmethylthio)or oxy)]alkylamino-2-methoxy-3,4-dioxocyclobutene. This latter compound can then in turn be reacted with an amine, $NHRR^6$ to yield a compound of formula (I) in which B is $NRR^6$. It goes without saying that other leaving groups of formula $YR^4$ can be used instead of methoxy in this reaction sequence.

Compounds according to the above formula wherein B is $NH_2$ and A is NCN can be prepared by a special reaction according to Flow Chart E.

FLOW CHART E.

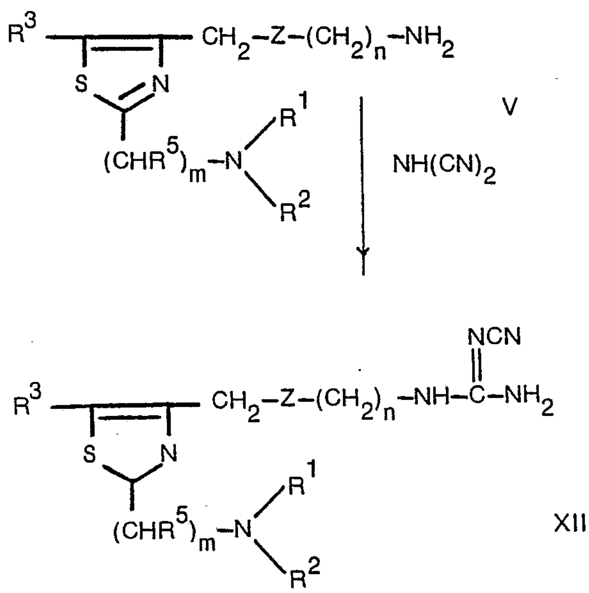


V

XII

wherein $R^1$, $R^2$, $R^3$, $R^5$, Z, n and m have then previously assigned value.

In this procedure, dicyanamide, usually generated in situ from one of its salts, preferably the sodium salt, is reacted with a thiazolyl primary amine (V) to yield the cyanoguanidine (XII) directly.

Compounds according to Formula (I) above wherein A is $N—CO—NH_2$; i.e., having a terminal group of the structure

$$\begin{array}{c} N—CO—NH_2 \\ \| \\ —NH—C—NHR, \end{array}$$

are prepared by hydration of the corresponding cyano compound,

$$\begin{array}{c} N—CN \\ \| \\ —NH—C—NHR, \end{array}$$

in dilute mineral acid such as, for example, dilute aqueous hydrochloric acid.

Finally, many of the compounds of this invention can be readily prepared via a carbodiimide intermediate as illustrated in Flow Chart F.

## FLOW CHART F

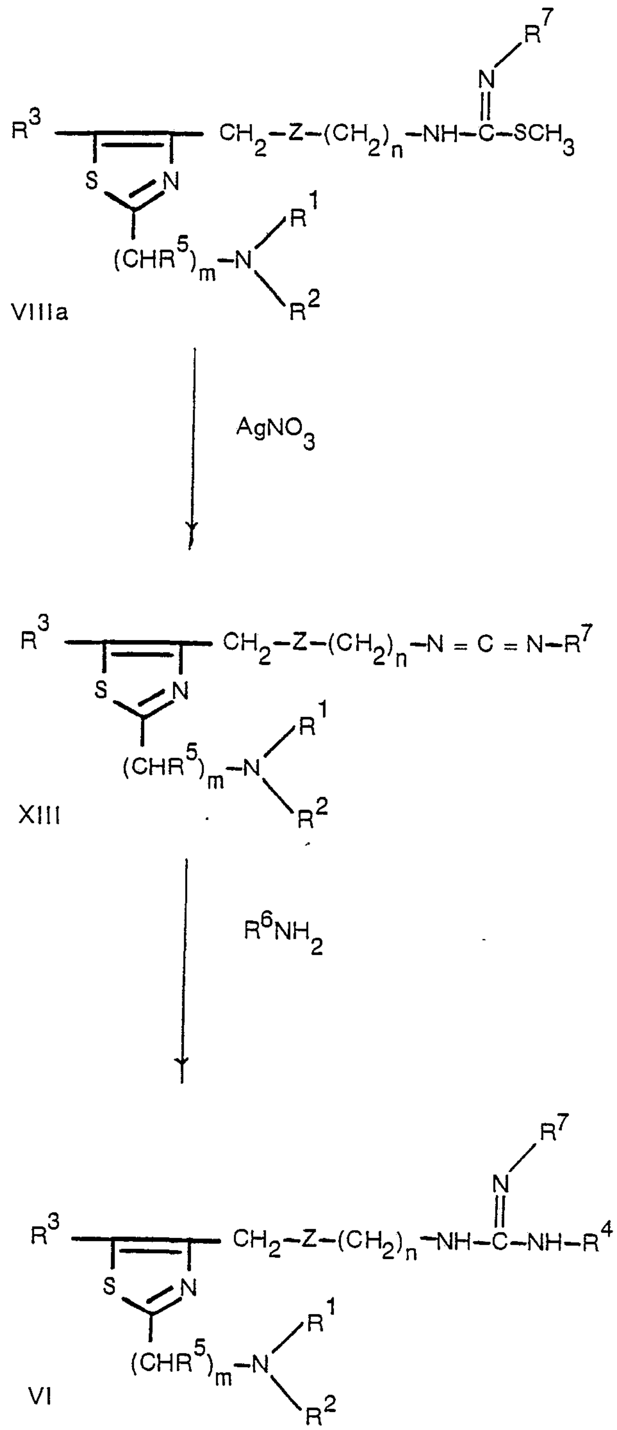


VIIIa

XIII

VI

wherein $R^1$—$R^4$, Z, n and m have their previously assigned value and $R^7$ is CN, $COC_{1-3}$ alkyl, $CO_2C_{1-3}$ alkyl, $SO_2$—aryl or $SO_2CH_3$ wherein aryl is phenyl, halophenyl, $(C_1$—$C_3)$alkylphenyl or $C_{1-3}$ alkoxyphenyl.

According to Flow Chart F, an isothiourea VIIIa (prepared by the procedure of Flow Chart C or equivalent procedure) is reacted with silver nitrate to prepare a carbodiimide (XIII), reaction of which with a primary amine, $R^6NH_2$, yields those compounds of this invention wherein A is NCN etc. (VI).

In the general case compounds of formula (I), where Q is C=A and B is $NHR^6$, can be prepared by reacting a compound of formula:

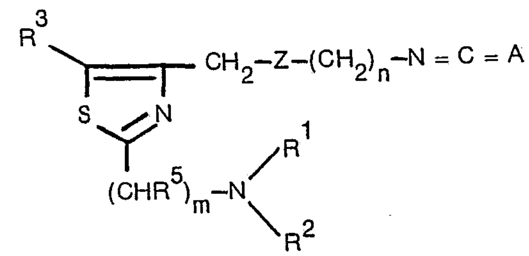

with an amine of formula $R^6NH_2$.

Compounds of formula (I) wherein Z is $CH_2$ and n is 1, 2 or 3, can be prepared by the procedure illustrated in Flow Chart G below.

FLOW CHART G

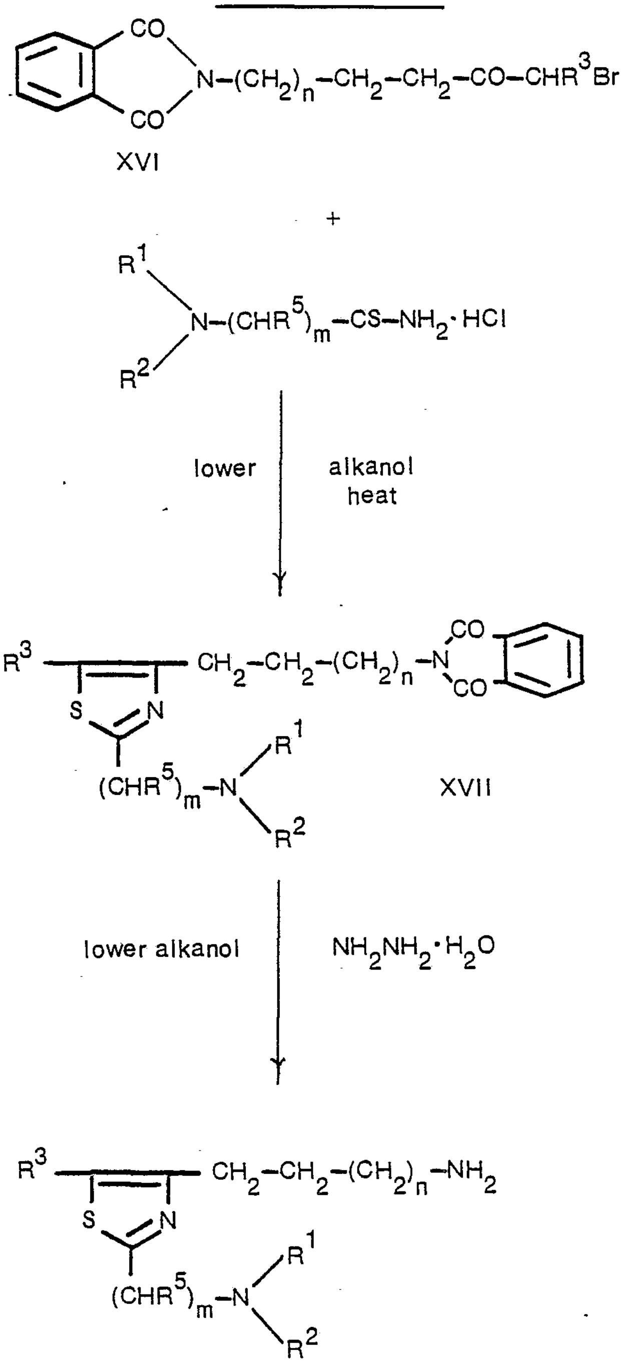


XVI

+

XVII

Vc

wherein $R^1$, $R^2$, $R^3$, $R^5$, n and m have their previously assigned meaning.

According to flow chart G, an omega (phthalimido)alkyl halomethyl ketone (XVI) is reacted with dimethylaminothioacetamide hydrochloride to produce a 2-aminoalkyl-5-permissibly substituted-4-omega-(phthalimido)alkylthiazole (XVII). The phthalimido group is removed by hydrolysis with hydrazine hydrate to produce the 4-(omega-aminoalkyl)thiazole ($V_c$). Alkaline hydrolysis with an alkali metal hydroxide followed by treatment with a dilute hydrochloric acid can also be used. This primary amine product (Vc) corresponds to the starting material ($V_a$) produced by flow chart A and can undergo each of the reactions set forth in Flow Charts B—F to produce the compounds of this invention wherein Z in formula (I) is $CH_2$.

In the above reaction schemes, the aminoalkyl group present at position 2 of the thiazole ring has been shown as carrying through each of the reaction steps essentially unchanged from the starting material employed (I in Flow Chart A). At times it is desirable to use certain alternate procedures in those instances where either $R^1$ or $R^2$ or both are hydrogen. For example, where $R^1$ is hydrogen but $R^2$ is alkyl, it is possible to use a benzyl protecting group through a given reaction scheme to the preparation of the hydroxymethyl derivative (IV) at which point the benzyl group can be removed by catalytic hydrogenation to give a secondary amine grouping $NHR^2$. Similarly, an acyl protecting group can be used such as a benzoyl group and this protecting group is removed by reduction to an alcohol during the lithium triethylborohydride reduction step by using excess borohydride. Similarly, if it is desired to have a primary alkylamino group at position 2 of the thiazole ring, a protecting group such as a phthalimido group can be utilized. In such instance, the starting material (I) would be one in which $R^1$ and $R^2$, when taken together with the nitrogen to which they are attached, form a phthalimido group. This grouping can be carried throughout the synthetic procedure until it is desired to remove it (after reaction of the ethylamine to form the side chain) by hydrolysis as with hydrazine. Such a protecting group would be particularly valuable in those instances where it is desired to utilize a 4-chloromethylthiazole as an intermediate.

An alternate procedure for preparing intermediates useful in the synthesis of compounds of this invention (I) starts with the reaction of dichloroacetone and a substituted aminothioacetamide. The use of the resulting 4-chloromethylthiazole where not more than one of $R^1$ and $R^2$ is H has been discussed previously in connection with Flow Chart A. However, this procedure is illustrated in Flow Chart H below and is particularly valuable in preparing the compounds of this invention wherein both $R^1$ and $R^2$ are H.

FLOW CHART H

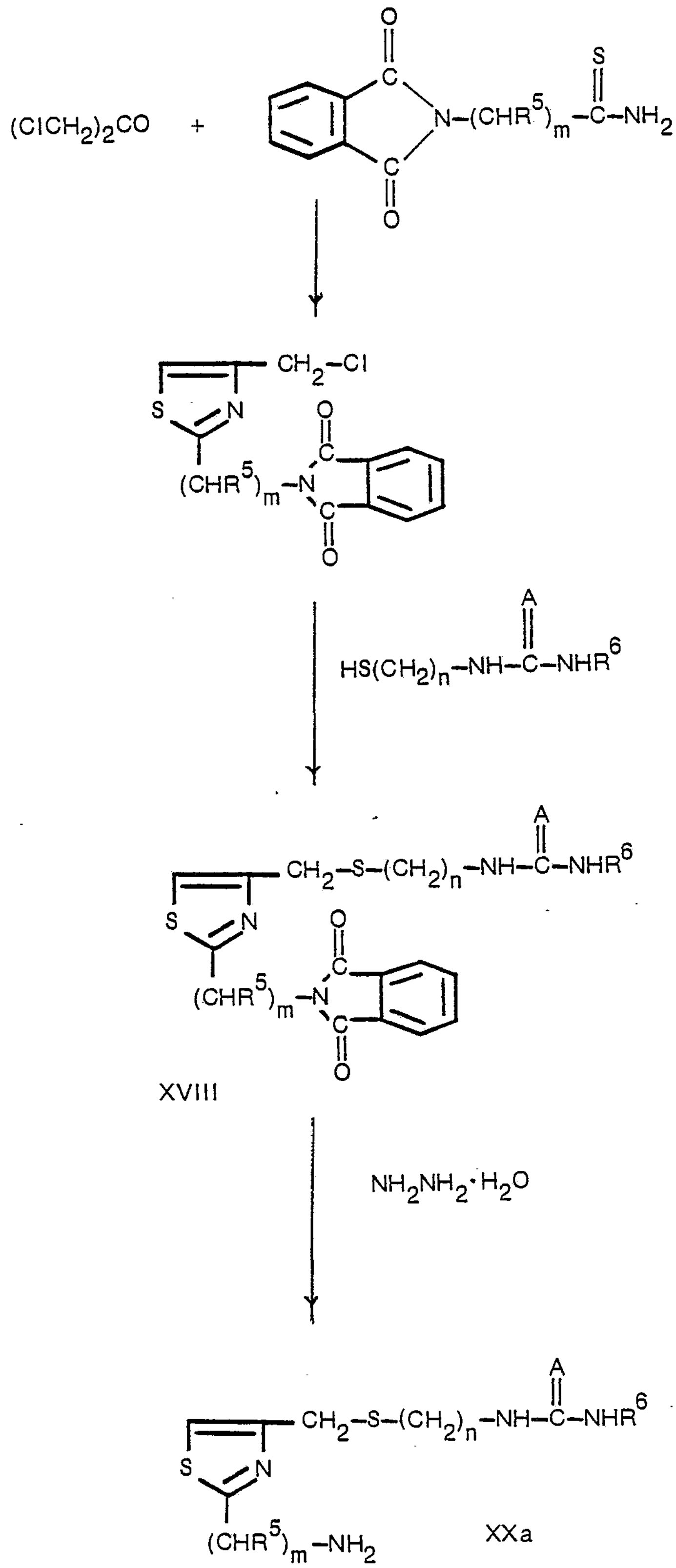


XVIII

XXa

wherein $R^6$, $R^5$, A, m and n have their previously assigned significances.

In Flow Chart H, a 2-(phthalimidoalkylthioacetamide) reacts with dichloro acetone, following the

procedure of *J. Am. Chem. Soc., 64,* 90 (1942), to yield a 2-(phthalimidoalkyl)-4-chloromethylthiazole (XVIII). Reaction of this intermediate with a cysteamine (or homocysteamine) derivative in which the amine group is substituted so as to form a desired terminal "amidine" provides a 2-(phthalimido)-4-thiazolyl derivative XVIII which can be hydrolyzed with hydrazine to yield the 2-aminoalkylthiazole derivative (I) in which $R^1$ and $R^2$ are H, Z is S, and A and $R^6$ have their previous scope).

This procedure constituted a general method of preparing compounds of formula (I) in which Z is sulfur. Thus, compounds of formula:

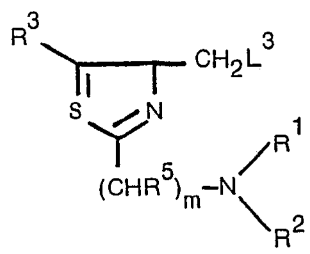

where $L^3$ is a good leaving group, such as halo, preferably chloro; ester, for example, tosyloxy or mesyloxy; or hydroxy, can be fused with the thiol of formula

$$HS(CH_2)_n—NH—CA—NRR^6.$$

This fusion reaction does not require a solvent and can be effected at temperatures from 60 to 130°C, preferably 90 to 100°C.

Those skilled in the art will appreciate that the guanidines and ethenediamines of the invention may exist in a number of tautomeric forms. It is to be clearly understood that, in any given case, although only one tautomeric form may be illustrated, the invention extends to all the other tautomeric forms.

This invention will now be further illustrated by the following Examples.

Preparation 1

Morpholinothioacetamide

A reaction mixture was prepared from 203 ml. each of triethylamine and pyridine plus 63 g. of morpholinoacetonitrile. Hydrogen sulfide was bubbled through the heated, stirred reaction mixture for about 2.5 hours. Stirring was continued overnight at ambient temperature. The next day, $H_2S$ was passed through the heated, stirred reaction mixture for an additional 1.5 hours. At this point, the solvents were evaporated *in vacuo* and the residue triturated with ether. The ether was discarded and the residue dissolved in ethanol. Crystalline morpholinothioacetamide precipitated and was separated by filtration. Treatment of the filtrate with alcoholic hydrogen chloride yielded morpholinothioacetamide hydrochloride melting in the range 64—80°C. See also *J.A.C.S., 72,* 2804 (1950).

Following the above procedure but using piperidinoacetonitrile in place of morpholinoacetonitrile, there was prepared piperidinothioacetamide hydrochloride melting at 166—168°C., after recrystallization from ethylacetate. See also *Helv. Chim. Act., 43,* 659 (1960).

Yield 35 g. from 62 g. of piperidinoacetonitrile starting material.

Following the above procedure using 100 g. of pyrrolidinoacetonitrile, there were obtained 68.4 g. of pyrrolidinothioacetamide hydrochloride (new) melting at about 195—197°C.

| | | | | |
|---|---|---|---|---|
| Analysis Calculated: | C, 39.88; | H, 7.25; | N, 15.50; | S, 17.74 |
| Found: | C, 39.66; | H, 6.99; | N, 15.76; | S, 17.84. |

Following the above procedure but using 49 g. of methylethylaminoacetonitrile, 200 ml. of triethylamine and 200 ml. of benzene, there was prepared N-methyl-N-ethylaminothioacetamide hydrochloride (new) melting at 115—117°C.

Example 1

Preparation of Ethyl 2-(Dimethylaminomethyl)-4-thiazolecarboxylate (intermediate)

A reaction mixture was prepared containing 15.5 g. of dimethylaminothioacetamide hydrochloride, 20.5 g. of ethyl bromopyruvate and 100 ml. of ethanol. The reaction mixture was heated to refluxing temperature for about four hours after which time the solvent was removed *in vacuo* in a rotary evaporator. The residue, containing ethyl 2-(dimethylaminomethyl)-4- thiazolecarboxylate formed in the above reaction, was dissolved in a mixture of ether and water. The aqueous layer was separated. The ether layer was extracted with an equal volume of water and then discarded. The two aqueous layers were combined and washed with ether. The ether layer was again discarded and the aqueous layer cooled to a temperature in the range of 0—5°C. Solid potassium carbonate was added until the aqueous layer gave a basic reaction to litmus. An oil separated comprising ethyl 2-(dimethylaminomethyl)-4-thiazolecarboxylate free base. The

oily layer was extracted with ether and the ether extract separated and dried. The ether was removed by evaporation in vacuo. The resulting residue was purified by gradient high pressure liquid chromatography over silica using a tolueneethyl acetate eluant. Ethyl 2-(dimethylaminomethyl)-4-thiazolecarboxylate thus obtained had the following physical characteristics:

| | | | | |
|---|---|---|---|---|
| Analysis Calculated: | C, 50.45; | H, 6.59; | N, 13.07; | S, 14.96 |
| Found: | C, 50.13; | H, 6.39; | N, 12.89; | S, 15.04. |

The nmr spectrum in $CDCl_3$ (TMS internal standard) gave the following signals (δ): 1.43 (triplet, 3H), 2.40 (singlet, 6H), 3.87 (singlet, 2H), 4.47 (quartet, 2H), 8.20 (singlet, 1H).

Following the above procedure, a solution containing 20.4 g. of ethyl bromopyruvate and 20.8 g. of N-methyl-N-benzoyl thioacetamide in 100 ml. of ethanol was heated to refluxing temperature for about 4 hours. The solvent was removed by evaporation *in vacuo* and the resulting residue dissolved in 65 ml. of 4.5N aqueous hydrochloric acid. The aqueous acidic layer was extracted with ether and the ether extract discarded. 11.5 g. of sodium carbonate were added to the aqueous layer. Ethyl 2-(methylbenzoylaminomethyl)-4-thiazolecarboxylate formed in the above reaction, being insoluble in the solution, separated and was extracted into ether. The ether extract was separated and dried. Evaporation of the ether yielded 20.2 g. of ethyl 2-(methylbenzoylaminomethyl)-4-thiazolecarboxylate melting at about 151.5—153.5°C. after crystallization from ethyl acetate.

| | | | |
|---|---|---|---|
| Analysis Calculated: | C, 59.19; | H, 5.30; | N, 9.20; |
| Found: | C, 58.98; | H, 5.25; | N, 8.90. |

The nmr spectrum in $CDCl_3$ (TMS internal standard) gave the following signals (δ): 1.42 (triplet, 3H), 3.07 (singlet, 3H), 4.41 quartet, 2H), 4.98 (singlet, 2H), 7.40 (apparent singlet, 5H), 8.16 (singlet, 1H).

Example 2

Preparation of 2-(Dimethylaminomethyl)-4-thiazolemethanol (intermediate)

A solution of 12.5 g. of ethyl 2-(dimethylaminomethyl)-4-thiazolecarboxylate dissolved in about 35 ml. of anhydrous tetrahydrofuran was prepared and then cooled to about 0°C. under a nitrogen atmosphere. About 130 ml. of a 1 molar solution of lithium triethylborohydride in THF was added in dropwise fashion while maintaining the temperature in the range 0—5°C. The reaction mixture was stirred for about two hours after which time 36 ml. of 6N aqueous hydrochloric acid were added while maintaining the temperature in the range −3°C. to 0°C. The volatile constituents were removed *in vacuo* on a rotary evaporator. Water was added to the resulting residue and again the volatile constituents were removed. Water was again added to the residue and the aqueous mixture extracted several times with ether. The ether extracts were separated and discarded. The aqueous solution was then chilled and made basic by the addition of solid potassium carbonate. The resulting alkaline aqueous mixture was extracted with ethyl acetate. 2-(Dimethylaminomethyl)-4-thiazolemethanol, being insoluble in the basic solution, separated and was extracted with several portions of ethyl acetate. The ethyl acetate extracts were combined, and the combined extracts washed with saturated aqueous sodium chloride and then dried. The ethyl acetate was removed by evaporation. The residue consisting of a brown oil weighing about 7.7 g comprised 2-(dimethylaminomethyl)-4-thiazolemethanol formed in the above reaction having the following physical and chemical characteristics.

| | | | |
|---|---|---|---|
| Analysis Calculated: | C, 48.81; | H, 7.02; | N, 15.26 |
| Found: | C, 48.71; | H, 6.77; | N, 15.85. |

The nmr spectrum in $CDCl_3$ (TMS internal standard) gave the following signals (δ): 2.33 (singlet, 6H), 3.74 (singlet, 2H), 4.32 (singlet, 1H), 4.72 (singlet, 2H), 7.15 (singlet, 1H).

Boiling point = 102°C. at 0.5 torr.

Following the above procedure, 22.5 g. of ethyl N-methyl-N-benzoyl 2-aminomethyl-4-thiazolecarboxylate were dissolved in 125 ml. of dry THF under a nitrogen atmosphere. 320 ml. of a 1M $LiEt_3BH$ in THF was added. (Excess borohydride was required over the amount in the above example because of the necessity of reducing both the ethyl ester group to a hydroxymethyl group and of removing the benzoyl group as benzyl alcohol leaving a secondary amine). The reaction mixture was worked up in accordance with the above procedure by decomposition with 6N aqueous hydrochloric acid and water. The residue remaining after the volatile constituents had been removed was a thick oil which was taken up in a little water and 60 ml. of ether. 1 ml. of 12N aqueous hydrochloric acid was added, thus making the aqueous phase strongly acidic. The ether layer was separated and the aqueous layer extracted five more times with equal portions of ether. The ether extracts were discarded. The water layer was separated and the water removed by evaporation *in vacuo*. The acidic residue was made strongly basic (while being cooled) with 50% aqueous sodium hydroxide (6 grams in 6 ml. of water). 2-Methylaminomethyl-4-thiazolemethanol produced by the above series of reactions was insoluble in the alkaline layer and separated. The compound was taken up in ethyl acetate using a continuous extractor. Removal of the solvent left a tannish oily

residue weighing 10.7 grams comprising 2-methylaminomethyl-4-thiazolemethanol. The compound was converted to the dihydrochloride salt by standard laboratory procedures.

Alternatively, a mixture of 2.14 g of ethyl 2-dimethylaminomethyl-4-thiazolecarboxylate and 0.38 g of sodium borohydride in 20 ml. of isopropanol was heated with stirring at reflux temperature for about 14 hours. The reaction mixture was cooled, and 2 ml. of water were added carefully followed by 4 ml. of 5N aqueous hydrochloric acid. The volatile constituents were removed by evaporation. Methanol (10 ml.) was added and the mixture heated to refluxing temperature for about one hour. Methanol was removed by evaporation and the residual solids digested in 10 ml. of isopropanol on the steam bath. The isopropanol solution was separated by decantation and the solids reextracted with 10 ml. of isopropanol. The isopropanol solutions and extracts were combined and the combined solution filtered while hot to remove insoluble material. The filtrate was chilled and a crystalline solid appeared which separated and was recovered by filtration. Recrystallization of the filter cake from isopropanol gave 1.73 g of 2-(dimethylaminomethyl)-4-thiazole methanol hydrochloride melting at 153—154°C.

| | | | | |
|---|---|---|---|---|
| Analysis: Calculated: | C, 40.28; | H, 6.28; | Cl, 16.99; | N, 13.42 |
| Found: | C, 40.38; | H, 5.04; | Cl, 17.24; | N, 13.12. |

The methanols produced by the process of this example are readily converted to the corresponding thiazole methyl chlorides according to the following procedure: A suspension was prepared from 1.05 grams of 2-(dimethylaminomethyl)-4-thiazole methanol hydrochloride and 15 ml. of chloroform. Thionyl chloride (2.50 g) was added and the resulting mixture was stirred at reflux temperature for about 2.75 hours. Any volatile constituents including excess thionyl chloride were removed by evaporation. The residue was suspended in chloroform and the chloroform removed by evaporation. The residue was then recrystallized from a methanol-ethyl acetate solvent mixture to yield 2-(dimethylaminomethyl)-4-thiazolylmethylchloride hydrochloride melting at 136—8°C.

| | | | | |
|---|---|---|---|---|
| Analysis: Calculated: | C, 37.01; | H, 5.32; | Cl, 31.21; | N, 12.33 |
| Found: | C, 37.13; | H, 5.06; | Cl, 31.41; | N, 12.36. |

## Example 3

Preparation of 2-([2-(Dimethylaminomethyl)-4-thiazolyl]methylthio)ethylamine (intermediate)

A reaction mixture was prepared from 18.8 g. of 2-dimethylaminomethyl-4-thiazolemethanol, 12.8 g. of 2-aminoethanethiol hydrochloride (cysteamine hydrochloride) and 160 ml. of 48% aqueous hydrobromic acid. The reaction mixture was stirred at about 100°C. for about 11 hours. The volatile constituents were removed *in vacuo* on a rotary evaporator. Water was added and the volatile constituents again removed by evaporation. The resulting residue, comprising 2-([2-(dimethylaminomethyl)-4-thiazolyl]methylthio)-ethylamine trihydrobromide formed in the above reaction, was dissolved in ethanol. The ethanol was evaporated and the resulting residue again dissolved in ethanol. Evaporation of the ethanol yielded a hygroscopic residue which was recrystallized from a methanol-ethyl acetate solvent mixture. 2-([2-(Dimethylaminomethyl)-4-thiazolyl]methylthio)ethylamine trihydrobromide thus prepared had the following physical and chemical characteristics:

| | | | | | |
|---|---|---|---|---|---|
| Analysis: Calculated: | C, 22.80; | H, 4.25; | Br, 50.56; | N, 8.86; | S, 13.53 |
| Found: | C, 23.02; | H, 4.31; | Br, 50.64; | N, 8.80; | S, 13.60. |

The nmr spectrum in $DMSOd_6$ (TMS internal standard) gave the following signals (δ): 2.55—3.2 (multiplet, 4H), 2.84 (singlet, 6H), 3.92 (singlet, 2H), 4.74 (singlet, 2H), 7.2—7.7 (broad, 1H), 7.94 (singlet, 1H), 7.92 (broad, 3H), 10.22 (broad, 1H).

Following the above procedure, 10.1 millimoles of 2-(methylaminomethyl)-4-thiazolemethanol dihydrochloride, 1.15 g. of cysteamine hydrochloride and 15 ml. of 48% aqueous hydrobromic acid were stirred at about 100°C. for about 7.5 hours. Water and hydrobromic acid were removed on a rotary evaporator and the resulting residue comprising 2-([2-(methylaminomethyl)-4-thiazolyl]methylthio)-ethylamine trihydrobromide formed in the above reaction was dissolved in water and the water removed by evaporation. The residue was again taken up in water and the water removed by evaporation. The residue was then dissolved in a small volume of water and a solution of 5.5 g. of potassium carbonate in 15 ml. of water was added. The resulting alkaline solution was evaporated to dryness. The resulting residue, comprising the free base of 2-([2-(methylaminomethyl)-4-thiazolyl]methylthio)ethylamine, was slurried with ethanol and the ethanol separated and removed by evaporation. The residue was twice slurried with isopropanol. The residue was next extracted with boiling isopropanol several times and the combined isopropanol extracts combined and filtered. Removal of the isopropanol yielded a yellow oil. The yellow oil was dissolved in chloroform and the chloroform solution filtered. Chloroform was evaporated from the filtrate to yield 1.59 g. of a yellow oil comprising 2-([2-(methylaminomethyl)-4-thiazolyl]methylthio)-ethylamine. The compound had the following physical characteristics:

The nmr spectrum is $CDCl_3$ (TMS internal standard) gave the following signals (δ): 1.53 (overlapping

singlets, 3H), 2.53 (singlet, 3H), 2.62 (triplet, 2H), 2.86 (triplet, 2H), 3.81 (singlet, 2H), 4.04 (singlet, 2H), 7.04 (singlet, 1H).

The above primary amine can be prepared by an alternate procedure involving the fusion of a 2-dialkylaminoalkyl-4-isothiazolylmethylchloride acid addition salt with an acid addition salt of cysteamine (or homocysteamine). This alternate procedure is illustrated below.

2-(Dimethylaminomethyl)-4-thiazolylmethylchloride hydrochloride (1.92 g.) and cysteamine hydrochloride (0.96 g.) were intimately mixed and the mixture heated slowly under anhydrous conditions to about 100°C over a period of one hour. The reaction mixture was then heated in the range 104—110°C for a period of 6 hours at which time the reaction was substantially complete as determined by tlc [95:5 ethanol-$NH_4OH$ (.88 sp. gr.)]. The reaction mixture was cooled and the cooled melt dissolved in a minimal amount of water. The solution was transferred to a rotary evaporator and the water removed. The resulting residue solidified and the solid was recrystallized from a methanol-ethyl acetate solvent mixture. Hygroscopic crystals of 2-([2-(dimethylaminomethyl)-4-thiazolyl]methylthio)ethyl amine trihydrochloride thus produced melted at about 165—72°C with evolution of HCl.

| | | | | | |
|---|---|---|---|---|---|
| Analysis Calculated: | C, 31.72; | H, 5.91; | Cl, 31.21; | N, 12.33; | S, 18.82 |
| Found: | C, 31.63; | H, 6.15; | Cl, 31.34; | N, 12.62; | S, 18.63. |

Example 4

Preparation of N-methyl-N'-2-([2-(dimethylaminomethyl)-4-thiazolyl]methylthio)ethyl-N''-cyanoguanidine

A solution was prepared from 3.07 g. of dimethyl cyanodithioimidocarbonate and 35 ml. of ethanol. A second solution containing 4.62 g. of 2-([2-(dimethylaminomethyl)-4-thiazolyl]methylthio)ethylamine in 50 ml. of ethanol was added in dropwise fashion with stirring to the first solution over a period of about 1.5 hours. The resulting reaction mixture was stirred for an additional 20 hours after which time the volatile constituents were removed in a rotary evaporator. Chromatography of the residue over silica by gradient elution using ethyl acetate containing increasing quantities of methanol as the eluant yielded fractions containing methyl N-2-([2-(dimethylaminomethyl)-4-thiazolyl]methylthio)ethyl-N'-cyanocarbamidothioate, formed in the above reaction. These fractions were combined and the solvent removed from the combined fractions in a rotary evaporator. The residue weighed 4.8 g. and, after recrystallization from carbon tetrachloride, melted at about 75—77°C.

| | | | | |
|---|---|---|---|---|
| Analysis Calculated: | C, 43.74; | H, 5.81; | N, 21.25; | S, 29.19 |
| Found: | C, 43.46; | H, 5.71; | N, 20.98; | S, 29.15. |

2.52 g. of the above thioester were dissolved in 12 ml. of methanol. 30 ml. of a 35% solution of methylamine (w/w) in ethanol was added with stirring. After five hours, the solvent and excess amine were removed by evaporation on a rotary evaporator. The residue was purified by chromatography over silica using gradient elution with an ethyl acetate-methanol solvent mixture as the eluant. Fractions containing N-methyl-N'-2-([2-(dimethylaminomethyl)-4-thiazolyl)]methylthio)ethyl-N''-cyanoguanidine formed in the above reaction were combined to yield 1.86 g. of a glassy residue upon evaporation of the solvent.

| | | | |
|---|---|---|---|
| Analysis Calculated: | C, 46.13; | H, 6.45; | N, 26.90 |
| Found: | C, 46.43; | H, 6.39; | N, 26.85. |

The nmr spectrum in $CDCl_3$ (TMS internal standard) shows the following signals (δ): 2.34 (singlet, 6H); 2.72 (triplet, 2H); 2.84 (doublet, 3H); 3.42 (multiplet, 2H); 3.74 (singlet, 2H); 3.82 (singlet, 2H); 6.08 (multiplet, 1H); 6.22 (multiplet, 1H); 7.10 (singlet, 1H).

Following the above procedure, but substituting ethylamine for methylamine in the reaction with N-2-([2-(dimethylaminomethyl)-4-thiazolyl]methylthio)ethyl-N'-cyanocarbamidothioate, N-ethyl-N'-2-([2-(dimethylaminomethyl)-4-thiazolyl]methylthio)ethyl-N''-cyanoguanidine was prepared.

| | | | | |
|---|---|---|---|---|
| Analysis Calculated for $C_{13}H_{22}N_6S_2$: | C, 47.82; | H, 6.79; | N, 25.74; | S, 19.64 |
| Found: | C, 48.05; | H, 7.01; | N, 25.51; | S, 19.33. |

The nmr in $CDCl_3$ (TMS internal standard) shows the following peaks (δ): 1.22 (triplet, 3H), 2.34 (singlet, 6H), 2.72 (triplet, 2H), 3.1—3.55 (multiplets unresolved, 4H), 3.74 (singlet, 2H), 3.82 (singlet, 2H), 5.7 (broad, 1H), 6.0 (broad, 1H), 7.08 (singlet, 1H).

Example 5

Preparation of N-2-([2-(dimethylaminomethyl)-4-thiazolyl]methylthio)ethyl-N'-methyl 2-nitro-1,1-ethenediamine.

A quantity of 2-([2-(dimethylaminomethyl)-4-thiazolyl)]methylthio)ethylamine trihydrobromide prepared from 50 g. of 2-(dimethylaminomethyl)-4-thiazolylmethanol by the procedure of Example 3 were dissolved in 150 ml. of water. A solution of 125 g. of potassium carbonate and 150 ml. of water was carefully added thereto. The water was removed by evaporation *in vacuo*. The resulting alkaline residue

was triturated with ethanol and isopropanol and the alkanol removed therefrom by evaporation. The resulting residue was extracted several times with hot isopropanol and the isopropanol extracts were filtered to remove inorganic salts. Evaporation of the solvent from the filtrate yielded a residue which was dissolved in chloroform and filtered. The chloroform was removed from the filtrate on a rotary evaporator. The resulting residue, comprising the free base of 2-([2-(dimethylaminomethyl)-4-thiazolyl]methylthio)-ethylamine, was dissolved in 250 ml. of water. This solution was added to a stirred suspension of 40.7 g. of N-methyl-1-methylthio-2-nitroethyleneamine (prepared according to the procedure of Belgian Patent 859,388) at 50°C. The solution was stirred at the same temperature for about 4 hours after the addition had been completed. Water was then removed by evaporation *in vacuo* on a rotary evaporator. The resulting residue was dissolved in ethanol and the solvent removed by evaporation. The residue was crystallized from an ethanol-acetonitrile solvent mixture and recrystallized from ethanol-ethyl acetate solvent mixture to yield 49.5 g. of N-methyl-N'-2-([2-(dimethylaminomethyl)-4-thiazolyl]methylthio)ethyl 2-nitro-1,1-ethenediamine melting at about 130—132°C.

| | | | | |
|---|---|---|---|---|
| Analysis Calculated: | C, 43.48; | H, 6.39; | N, 21.13; | O, 9.65 |
| Found: | C, 43.66; | H, 6.40; | N, 21.14; | O, 9.46. |

The nmr spectrum in $CDCl_3$ (TMS internal standard) gave the following signals (δ): 2.24 (singlet, 6H), 2.68 (triplet, 2H), 2.74 (singlet, 3H), 3.34 (multiplet, 2H), 3.70 (singlet, 2H), 4.84 (singlet, 2H), 6.46 (singlet, 1H), 7.16 (broad, 1H), 7.40 (singlet, 1H), 9.96 (broad, 1H).

Following the above procedure, 2-([2-(methylaminomethyl)-4-thiazolyl]methylthio)ethylamine and N-methyl-1-methylthio-2-nitroethyleneamine were reacted in water solution. The reaction was worked up and the product isolated by the above procedure to yield N-methyl-N'-2-([2-(methylaminomethyl)-4-thiazolyl]methylthio)ethyl 2-nitro-1,1-ethenediamine melting at 105—107°C. after recrystallization from acetonitrile followed by recrystallization from ethanol.

| | | | | |
|---|---|---|---|---|
| Analysis Calculated: | C, 41.62; | H, 6.03; | N, 22.06; | O, 10.08 |
| Found: | C, 41.79; | H, 6.10; | N, 21.80; | O, 10.28. |

Following the above procedure, 2-([2-(dimethylaminomethyl)-4-thiazolyl)methylthio)ethylamine was caused to react with N-ethyl-1-methylthio-2-nitroethyleneamine to yield N-ethyl-N'-2-([2-(dimethylaminomethyl)-4-thiazolyl]methylthio)ethyl 2-nitro-1,1-ethenediamine melting at about 89—90°C.

| | | | | |
|---|---|---|---|---|
| Analysis Calculated for $C_{13}H_{23}N_5O_2S_2$: | C, 45.19; | H, 6.71; | N, 20.27; | O, 9.26 |
| Found: | C, 45.32; | H, 6.70; | N, 20.44; | O, 9.49. |

## Example 6

Preparation of N-methyl-N'-2-([2-(dimethylaminomethyl)-4-thiazolyl]methylthio)ethylthiourea

A solution was prepared containing 0.80 g. of 2-([2-(dimethylaminomethyl)-4-thiazolyl]methylthio)-ethylamine and 0.29 g. of methylisothiocyanate in 10 ml. of ethanol. The solution was stirred at room temperature for about 17 hours after which time the solvent was removed by evaporation *in vacuo*. The residual gum, comprising N-methyl-N'-2-([2-(dimethylaminomethyl)-4-thiazolyl]methylthio)ethylthiourea formed in the above reaction, was purified by chromatography over silica using a gradient elution technique with ethyl acetate containing increasing quantities of methanol as the eluant. Fractions containing the above compound were combined and the solvent evaporated therefrom *in vacuo*, leaving as a residue 0.83 g. of a glassy solid.

| | | | | |
|---|---|---|---|---|
| Analysis Calculated: | C, 43.39; | H, 6.62; | N, 18.40; | S, 31.59 |
| Found: | C, 43.62; | H, 6.49; | N, 18.15; | S, 31.70. |

The nmr spectrum in $CDCl_3$ (TMS internal standard) gave the following signals (δ): 2.34 (singlet, 6H), 2.80 (triplet, 2H), 3.00 (doublet, 3H), 3.74 (singlet, 2H), 3.82 (singlet, 2H), 3.6—3.9 (multiplet, 2H), 6.9 (broad, 1H), 7.08 (singlet, 1H), 7.2 (broad, 1H).

Following the above reaction sequence, a solution of 0.93 g of 2-[2-(dimethylaminomethyl)-4-thiazolyl]methylthio)ethylamine in 10 ml. of anhydrous acetonitrile was prepared. A solution of 0.22 ml. of methyl isocyanate in 5 ml. of anhydrous acetonitrile was added thereto. The reaction was stirred at ambient temperature for about 2.5 hours at which time the solvent was removed by evaporation. The resulting residue slowly crystallized and was recrystallized from cyclohexane to yield about 0.97 g of N-methyl-N'-2-([2-(dimethylaminomethyl)-4-thiazolyl]methylthio)ethylurea melting at about 56—59°C.

| | | | | |
|---|---|---|---|---|
| Analysis: Calculated: | C, 45.81; | H, 6.99; | N, 19.42; | O, 5.55 |
| Found: | C, 46.03; | H, 6.66; | N, 19.41; | O, 5.71. |

Example 7

Preparation of N-methyl-N′-2-([2-(dimethylaminomethyl)-4-thiazolyl]methylthio)ethyl-N″-p-tolylsulfonylguanidine

A suspension was prepared by adding 1.35 g. of dimethyl p-toluenesulfonylimidodithiocarbonate to 10 ml. of ethanol. While the suspension was being stirred at ambient temperature, a solution of 1.16 g. of 2-([2-(dimethylaminomethyl)-4-thiazolyl]methylthio)ethylamine in 10 ml. of ethanol was added over a 15 minute period. The reaction mixture was stirred for 2.5 hours after the addition had been completed. The solvent was then removed by evaporation *in vacuo*. The resulting residue was mixed with 20 ml. of a 35% solution (w/w) of ethanolic methylamine. This reaction mixture was stirred for about 15 hours after which time the solvent and other volatile constituents were removed by evaporation *in vacuo*. The residue was chromatographed over silica using a gradient elution technique employing ethyl acetate containing increasing quantities of methanol as the eluant. Fractions containing N-methyl-N′-2-([2-(dimethylaminomethyl)-4-thiazolyl]methylthio)ethyl-N″-p-tolylsulfonylguanidine formed in the above reaction were combined and the solvent removed therefrom to leave as a residue 1.9 g. of a glass.

| | | | | | |
|---|---|---|---|---|---|
| Analysis Calculated: | C, 48.95; | H, 6.16; | N, 15.86; | O, 7.25; | S, 21.78 |
| Found: | C, 49.25; | H, 6.27; | N, 16.10; | O, 7.45; | S, 21.62. |

The nmr spectrum in $CDCl_3$ (TMS internal standard) gave the following signals (δ): 2.34 (singlet, 6H), 2.38 (singlet, 3H), 2.62 (triplet, 2H), 2.80 (doublet, 3H), 3.35—3.55 (multiplet, greater than 2H), 3.72 (singlet, 2H), 3.74 (singlet, 2H), 7.06 (singlet, 1H), 7.18 (doublet, 2H), 7.70 (doublet, 2H).

The above procedure was repeated using dimethyl methanesulfonylimidodithiocarbonate as a starting material. N-methyl-N′-2-([2-(dimethylaminomethyl)-4-thiazolyl]methylthio)ethyl-N″-methanesulfonylguanidine was obtained, m.p. 95—97°C after recrystallization from ethylacetate.

| | | | |
|---|---|---|---|
| Analysis: Calculated: | C, 39.43; | H, 6.34; | O, 8.75; |
| Found: | C, 39.71; | H, 6.19; | O, 8.72. |

Example 8

Preparation of N-methyl-N′-2-([2-(dimethylaminomethyl)-4-thiazolyl]methylthio)ethyl-N″-nitroguanidine

A reaction mixture was prepared containing 1.2 g. of 2-([2-(dimethylaminomethyl)-4-thiazolyl]methylthio)ethylamine, 0.77 g. of S-methyl-N-methyl-N′-nitroisothiourea and 10 ml. of methanol. The reaction mixture was heated under reflux for 4.25 hours, after which time the solvent was removed by evaporation. The partially solid residue was chromatographed over silica using a gradient elution technique employing ethyl acetate containing increasing quantities of methanol as the eluant. Fractions shown by TLC to contain N-methyl-N′-2-([2-(dimethylaminomethyl)-4-thiazolyl]methylthio)ethyl-N″-nitroguanidine formed in the above reaction were pooled and the solvent removed from the pooled fractions. Trituration of the resulting residue with ether yielded a crystalline solid which, upon recrystallization from a methanol-ethyl acetate solvent mixture, yielded 0.83 g. of crystals melting at about 86.5—88°C.

| | | | | | |
|---|---|---|---|---|---|
| Analysis Calculated: | C, 39.74; | H, 6.06; | N, 25.28; | O, 9.62; | S, 19.29 |
| Found: | C, 39.92; | H, 5.89; | N, 25.15; | O, 9.38; | S, 19.49. |

Following the above procedure, 2-([2-dimethylaminomethyl)-4-thiazolyl]methylthio)ethylamine was reacted with S-methyl N-nitroisothiourea to yield N-2-([2-dimethylaminomethyl)-4-thiazolyl]methylthio)ethyl-N′-nitroguanidine which melted at 104—105.5°C. after recrystallization from ethyl acetate. The compound was purified by chromatography over silica using an ethyl acetate/methanol solvent mixture as the eluent.

| | | | | |
|---|---|---|---|---|
| Analysis: Calculated: | C, 37.72; | H, 5.70; | N, 26.39; | O, 10.05; |
| Found: | C, 37.88; | H, 5.41; | N, 26.10; | O, 10.34. |

Example 9

Preparation of N-methyl-N′-2-([2-dimethylaminomethyl)-5-methyl-4-thiazolyl]methylthio)ethyl 2-nitro-1,1-ethenediamine

Following the procedure of Example 1, a reaction mixture containing 33.88 g. of ethyl 2-oxo-3-bromobutyrate [prepared by the procedure of Siefert et al., *Helv. Chim. Acta, 33,* 725 (1950)], 21.52 g. of dimethylaminothioacetamide hydrochloride and 100 ml. of anhydrous ethanol was stirred and heated to refluxing temperature for about 2.5 hours. The reaction mixture was allowed to remain at room temperature overnight after which time it was concentrated by evaporation *in vacuo*. 100 ml. of an ice-water mixture was added to the resulting residue and the aqueous layer extracted with ethyl acetate. The ethyl acetate layer was discarded. The aqueous layer was cooled and then made basic (pH = 11) with 2N aqueous sodium hydroxide. The resulting alkaline layer was extracted several times with an equal volume of ethyl acetate and the ethyl acetate extracts were combined. The combined extracts were washed with water, with saturated aqueous sodium chloride, and were then dried. Concentration *in vacuo* provided a reddish

oil comprising ethyl 2-(dimethylaminomethyl)-5-methyl-4-thiazolecarboxylate. Yield = 21.2 g. (57%).

Two hundred milliliters of a one molar solution of lithium triethylborohydride in THF were cooled in an ice bath under a nitrogen atmosphere. A solution of 21.2 g. of ethyl 2-(dimethylaminomethyl)-5-methyl-4-thiazolecarboxylate in 60 ml. of THF was added in dropwise fashion over about 1.5 hours. The reaction mixture was maintained for an additional hour at 0°C. after which time it was decomposed by the cautious addition of 4 ml. of water plus 6 ml. of THF followed by 50 ml. of 6N aqueous hydrochloric acid. The resulting reaction mixture was concentrated *in vacuo* and the residue treated with water. Solid potassium carbonate was added to the aqueous mixture to pH = 11. The resulting aqueous alkaline mixture was extracted several times with equal volumes of ethyl acetate. The ethyl acetate extracts were separated and combined and the combined extracts dried. The ethyl acetate was removed therefrom *in vacuo* to leave, as a residue, 2-(dimethylaminomethyl)-5-methyl-4-thiazolemethanol; yield = 6.44 g. (44%).

Following the procedure of Example 3, a reaction mixture consisting of 6.4 g. of ethyl 2-(dimethylaminomethyl)-5-methyl-4-thiazolemethanol, 4.2 g. of cysteamine hydrochloride and 30 ml. of 48% aqueous hydrobromic acid was maintained at a temperature of about 100°C. under a nitrogen atmosphere for about 4 hours. The reaction mixture was cooled and the volatile constituents removed *in vacuo*. The resulting dark residue was twice triturated with ethanol and the ethanol removed by evaporation to remove residual HBr. The residue was then treated with 50 ml. of 5N aqueous sodium hydroxide. The alkaline layer was continuously extracted with ether over an 18 hour period. The ether extract was dried and the ether removed therefrom *in vacuo* to provide 2-([2-dimethylaminomethyl)-5-methyl-4-thiazolyl]methylthio)ethylamine prepared in the above reaction; yield = 1.38 g. The compound was a brown oil having the following physical characteristics.

The nmr spectrum in $CDCl_3$ (TMS internal standard) shows the following peaks (δ): 1.48 (singlet, 2H), 2.35 (singlet, 6H), 2.42 (singlet, 3H), 2.80 (multiplets, 4H), 3.72 (singlet, 2H), 3.80 (singlet, 2H).

Following the procedure of Example 5, a stirred solution of 1.38 g. of 2-([2-dimethylaminomethyl)-5-methyl-4-thiazolyl]methylthio)ethylamine in 10 ml. of methanol was treated with N-methyl-1-methylthio-2-nitroethyleneamine. The reaction mixture was kept at room temperature overnight by which time all solids had dissolved. Thin layer chromatography over silica using a 10:10:1 ethyl acetate-methanol-ammonium hydroxide solvent system indicated substantially a single product. The reaction mixture was concentrated by evaporation of the methanol and the resulting gummy yellow residue was triturated with several portions of cold ether, thus providing an off-white gum. Repeated trituration with cold 1,2-dimethoxyethane yielded N-2-([2-dimethylaminomethyl)-5-methyl-4-thiazolyl]methylthio)ethyl-N'-methyl-2-nitro-1,1-ethenediamine formed in the above reaction melting at about 104—106°C.

| | | | |
|---|---|---|---|
| Analysis: Calculated: | C, 45.19; | H, 6.71; | N, 20.27 |
| Found: | C, 45.54; | H, 6.47; | N, 19.60. |

The compound had the following physical characteristics:

The nmr spectrum in $CDCl_3$ (TMS internal standard) shows the following peaks (δ): 2.33 (singlet, 6H), 2.40 (singlet, 3H), 2.85 (multiplet, 2H), 2.97 (doublet, 3H), 3.48 (multiplet, 2H), 3.68 (singlet, 2H), 3.82 (singlet, 2H), 6.67 (singlet, 1H), 10.3 (broad, less than 2H).

Example 10

Preparation of N-4-[2-(dimethylaminomethyl)-4-thiazolyl]butyl-N'-methyl 2-nitro-1,1-ethenediamine

Following the procedure of Example 1, a stirred solution containing 3.2 g. of dimethylaminothioacetamide hydrochloride, and 6.48 g of bromomethyl 4-phthalimidobutyl ketone [prepared by the procedure of *Chem. Listy., 49,* 1385 (1955); *C.A., 50,* 5573c (1956)]; in 50 ml. of ethanol was heated at refluxing temperature for about 5 hours and was then cooled. Volatile constituents were removed by evaporation *in vacuo* leaving 2-(dimethylaminomethyl)-4-(phthalimido-1-butyl)thiazole as a semi-solid residue. The compound was utilized without further purification.

A solution was prepared containing the above product in 50 ml. of methanol. While the solution was being stirred, 2 ml. of 85% hydrazine hydrate were added and the resulting mixture heated to refluxing temperature for about 2 hours. At this point, an additional 2 ml. of 85% hydrazine hydrate were added and the heating continued for an additional two hours. The reaction mixture was then diluted with 4 volumes of water and the aqueous mixture made strongly basic with concentrated aqueous sodium hydroxide. The resulting alkaline layer was extracted continuously with ether for a 24 hour period. The ether extract was dried and the ether removed therefrom *in vacuo* to provide 4-[2-(dimethylaminomethyl)-4-thiazolyl]butylamine as a brown oil; weight = 1.81 g. (42% yield from bromoketone). Mass spectrum: m/e at 152, 138, 128, 112, 96, 79, 71, 58, 42, 30 and 15.

A solution of 1.1 g. of the primary amine produced in the preceding step in 15 ml. of methanol was stirred while a solution of 3.20 g. of N-methyl 1-methylthio-2-nitroethyleneamine in methanolic solution was added thereto. The reaction mixture was maintained at room temperature for about 24 hours during which time evolution of methylmercaptan was noted. The progress of the reaction was followed by TLC. After the reaction had gone to completion according to TLC analysis, the volatile constituents were removed *in vacuo* and the resulting residue dissolved in a 9:1 ethyl acetate-methanol solvent mixture. This solution was placed on 15 g. of Woelm silica and the chromatogram developed with the same solvent

mixture. Fractions shown by TLC to contain N-4-[2-(dimethylaminomethyl)-4-thiazolyl]butyl-N′-methyl 2-nitro-1,1-ethenediamine formed in the above reaction were combined and the solvent evaporated from the combined fractions *in vacuo* leaving a residual gum. Repeated trituration of this gum with small volumes of toluene followed by repeated recrystallization of the triturated solid from benzene provided off-white crystals melting at 97—99°C.

| | | | | |
|---|---|---|---|---|
| Analysis Calculated: | C, 49.82; | H, 7.40; | N, 22.35; | S, 10.23 |
| Found: | C, 49.56; | H, 7.25; | N, 22.12; | S, 9.95. |

The compound had the following peaks by mass spectral analysis: m/e at 236, 212, 194, 178, 153, 126, 112, 97, 85, 71, 58, 42, 32 and 15.

Example 11

Preparation of N-methyl-N′-2-([2-(morpholinomethyl)-4-thiazolyl]methylthio)ethyl 2-nitro-1,1-ethenediamine

Following the procedure of Example 1, morpholinothioacetamide hydrochloride was condensed with ethyl bromopyruvate to yield ethyl 2-(4-morpholinomethyl)-4-thiazolecarboxylate, melting at 129—130°C. after recrystallization from a methylene dichloride-ethyl acetate solvent mixture.

| | | | |
|---|---|---|---|
| Analysis Calculated: | C, 51.54; | H, 6.29; | N, 10.93. |
| Found: | C, 51.36; | H, 6.05; | N, 10.88. |

Following the procedure of Example 2, the above ester was reduced to the corresponding thiazolemethanol, 2-(4-morpholinomethyl)-4-thiazolemethanol, having an nmr spectrum in $CDCl_3$ (TMS internal standard) showing the following signals (δ):2.55 (multiplet, 4H), 3.35—3.90 (singlet plus multiplet, 6H), 4.70 (3H), 7.13 (singlet, 1H).

Reaction of the thiazolemethanol with cysteamine hydrochloride by the procedure of Example 5 yielded 2-([2-(4-morpholinomethyl)-4-thiazolyl]methylthio]ethylamine having the following physical characteristics:

nmr spectrum in $CDCl_3$ (TMS internal standard) showing the following signals (δ): 1.83 (singlet, 2H), 2.3—3.1 (multiplet, 8H), 3.4—3.9 (multiplet plus singlets, 8H), 7.03 (singlet, 1H).

Following the procedure of Example 5, the 2-[(4-thiazolyl)methylthio]ethylamine was reacted with N-methyl-1-methylthio-2-nitroethyleneamine to yield N-methyl-N′-2-([2-(4-morpholinomethyl)-4-thiazolyl]-methylthio]ethyl-2-nitro-1,1-ethenediamine melting at 151—153°C. after recrystallization from a methanol-ethyl acetate solvent mixture.

| | | | |
|---|---|---|---|
| Analysis Calculated: | C, 45.02; | H, 6.21; | N, 18.75. |
| Found: | C, 45.23; | H, 6.24; | N, 18.56. |

Example 12

Preparation of N-methyl-N′-2-([2-(1-pyrrolidinomethyl)-4-thiazolyl]methylthio)ethyl 2-nitro-1,1-ethenediamine

The same sequence of reactions as in Example 11 were carried out starting with pyrrolidinomethylthioacetamide hydrochloride to yield the following intermediates.

Ethyl 2-(1-pyrrolidino)-4-thiazolecarboxylate. M.P. = 81—81.5°C. after recrystallization from a tolueneethyl acetate solvent mixture.

Nmr spectrum in $CDCl_3$ (TMS internal standard) showed the following signals (δ): 1.40 (triplet 3H), 1.82 (multiplet, 4H), 2.70 (multiplet, 4H), 4.02 (singlet, 2H), 4.45 (quartet, 2H), 8.17 (singlet, 1H).

2-(1-pyrrolidinomethyl)-4-thiazolemethanol. Nmr spectrum in $CDCl_3$ (TMS internal standard) gave the following signals (δ): 1.77 (multiplet, 4H), 2.65 (multiplet, 4H), 3.92 (singlet, 2H), 4.72 (singlet, 3H), 7.15 (singlet, 1H).

2-([2-(1-pyrrolidinomethyl)-4-thiazolyl]methylthio)ethylamine trihydrobromide was crystallized from isopropanol.

The ethylamine obtained from the above hydrobromide was reacted with N-methyl-1-methylthio-2-nitroethyleneamine to yield N-methyl-N′-2-([2-(1-pyrrolidinomethyl)-4-thiazolyl]methylthio]ethyl 2-nitro-1,1-ethenediamine melting at 119—120°C. after recrystallization from a methanol-ethyl acetate solvent mixture.

| | | | |
|---|---|---|---|
| Analysis Calculated: | C, 47.04; | H, 6.49; | N, 19.59. |
| Found: | C, 46.81; | H, 6.55; | N, 19.04. |

Example 13

Preparation of N-methyl-N′-2-([2-(1-piperidinomethyl)-4-thiazolyl]methylthio)ethyl 2-nitro-1,1-ethenethiamine

Following the sequence of reactions of Example 11, the following intermediates were produced from

1-piperidinothioacetamide hydrochloride.

Ethyl 2-(1-piperidinomethyl)-4-thiazolecarboxylate melting at 95—97°C.

Nmr in $CDCl_3$ (TMS internal standard) gave the following signals (δ): 1.40 (triplet, 3H), 1.53 (multiplet, 6H), 2.53 (multiplet, 4H), 3.85 (singlet, 2H), 4.45 (quartet, 2H), 8.20 (singlet, 1H).

2-(1-piperidinomethyl)-4-thiazolemethanol having an nmr spectrum in $CDCl_3$(TMS internal standard) which gave the following signals (δ): 1.53 (multiplet, 6H), 2.47 (multiplet, 4H), 3.77 (singlet, 2H), 4.77 (singlet, >3H), 7.13 (singlet, 1H).

2-([2-(1-piperidinomethyl)-4-thiazolyl]methylthio)ethylamine trihydrobromide crystallized from isopropanol. The nmr spectrum in $DMSOd_6$ (TMS internal standard) showed the following signals (δ): 1.77 (multiplet, 6H), 2.6—3.8 (8H, multiplets), 3.97 (singlet, 2H), 4.80 (singlet, 2H), 7.80 (singlet, 1H), 8.12 (broad, 3H).

The primary amine obtained from the above salt was reacted with N-methyl 1-methylthio-2-nitroethyleneamine to yield N-methyl-N′-2-([2-(1-piperidinomethyl)-4-thiazolyl]methylthio)ethyl 2-nitro-1,1-ethenediamine melting at about 100—103°C.. after recrystallization from a methanol-ethyl acetate solvent mixture.

| | | | |
|---|---|---|---|
| Analysis Calculated: | C, 48.49; | H, 6.78; | N, 18.85 |
| Found: | C, 48.72; | H, 6.94; | N, 18.64. |

## Example 14

Preparation of N-methyl-N′-2-([2-(methylethylaminomethyl)-4-thiazolyl]methylthio)ethyl 2-nitro-1,1-ethenediamine.

Following the reaction sequence of Example 11 starting with the reaction of N-methyl-N-ethyl aminothioacetamide hydrochloride and ethyl bromopyruvate, the following intermediates were prepared:

Ethyl 2-(methylethylaminomethyl)-4-thiazolecarboxylate, a non-crystallizable oil

2-(methylethylaminomethyl)-4-thiazolemethanol having an nmr spectrum in $CDCl_3$ (TMS internal standard) showing the following signals (δ): 1.10 (triplet, 3H), 2.33 (singlet, 3H), 2.53 (quartet, 2H), 3.80 (singlet, 2H), 4.73 (singlet, 2H), 5.30 (singlet, 1H), 7.20 (singlet, 1H).

2-([2-(methylethylaminomethyl)-4-thiazolyl]methylthio)ethylamine having an nmr spectrum in $CDCl_3$ (TMS internal standard) showing the following signals (δ): 1.08 (triplet, 3H), 1.57 (singlet, 2H), 2.33 (singlet, 3H), 2.2—3.0 (multiplets, 6H), 3.78 (apparent singlet, 4H), 7.03 (singlet, 1H).

The above primary amine was reacted with N-methyl 1-methylthio-2-nitroethyleneamine to yield N-methyl-N′-2-([2-(methylethylaminomethyl)-4-thiazolyl]methylthio)ethyl 2-nitro-1,1-ethenediamine melting at 114—116°C. after recrystallization from a methanol-ethyl acetate solvent mixture.

| | | | |
|---|---|---|---|
| Analysis Calculated: | C, 45.19; | H, 6.71; | N, 20.27 |
| Found: | C, 45.48; | H, 6.80; | N, 19.98. |

## Example 15

Preparation of N-methyl-N′-2-([2-(dimethylaminomethyl)-4-thiazolyl]methylthio)ethyl-N″-aminocarbonylguanidine.

About 0.6 g. of N-methyl-N′-2-([2-(dimethylaminomethyl)-4-thiazolyl]methylthio)ethyl-N″-cyanoguanidine were dissolved in 8 ml. of 1.5N aqueous hydrochloric acid. The resulting solution was allowed to remain at ambient temperature for about four days. Volatile constituents were then removed by evaporation *in vacuo*. The resulting residue, comprising N-methyl-N′-2-([2-(dimethylaminomethyl)-4-thiazolyl]-methylthio)ethyl-N″-aminocarbonylguanidine dihydrochloride formed in the above reaction, was dissolved in ethanol and the ethanol removed by evaporation. The residue obtained thereby was recrystallized from isopropanol. The crystalline product was collected and digested with ethyl acetate. The ethyl acetate was removed by evaporation and the product obtained was crystallized from isopropanol to yield N-methyl-N′-2-([2-(dimethylaminomethyl)-4-thiazolyl]methylthio)ethyl-N″-aminocarbonylguanidine dihydrochloride melting at 156.5—159.5°C.

| | | | | |
|---|---|---|---|---|
| Analysis Calculated for $C_{12}H_{23}Cl_2N_6OS_2$: | C, 35.82; | H, 5.76; | Cl, 17.62; | O, 3.98 |
| Found: | C, 35.64; | H, 6.30; | Cl, 17.73; | O, 4.38. |

## Example 16

Preparation of N-(1-methylethyl)-N′-2-([2-(dimethylaminomethyl)-4-thiazolyl]methylthio)ethyl-N″-cyanoguanidine.

A reaction mixture was prepared from 357 mg of silver nitrate, 3 ml of isopropylamine and 12 ml of ethanol. To this stirred reaction mixture was added a solution of 658 mg of methyl N-2-([2-(dimethylaminomethyl)-4-thiazolyl]methylthio)ethyl-N′-cyanocarbamidothioate from Example 4 and 15 ml. of ethanol. The reaction mixture was stirred at ambient temperature for about 18 hours after which time a precipitate which formed was separated by filtration. The filter cake was washed with ethanol. Evaporation of the filtrate and the washings yielded a residue which was chromatographed over silica using a gradient elution technique. A solvent mixture containing 97.5% ethyl acetate and 2.5% methanol as the eluant eluted fractions

containing N-(1-methylethyl)-N'-2-([2-(dimethylaminomethyl)-4-thiazolyl]methylthio)ethyl-N''-cyanoguanidine. The compound was an oil.

| | | | | |
|---|---|---|---|---|
| Analysis: Calculated: | C, 49.38; | H, 7.10; | N, 24.68; | S, 18.83 |
| Found: | C, 49.08; | H, 7.19; | N, 24.77; | S, 18.62 |

($CDCl_3$): δ at 1.26 (d, 6H); 2.37 (s, 6H); 2.65 (trip, 2H); 3.45 (db, of trip, 2H); 3.76 (s, 2H); 3.83 (s, 2H); 5.23 (d, 1H); 5.81 (trip, 1H); 7.08 (s, 1H); 3.5—4.1 (multiplet, 1H)

Example 17

Preparation of N-2-([2-(dimethylaminomethyl)-4-thiazolyl]methylthio)ethyl-N'-(2-methoxyethyl)-2-nitro-1,1-ethenediamine.

A reaction mixture was prepared containing 2.31 g of 2-([2-(dimethylaminomethyl)-4-thiazolyl]methylthio)ethylamine, and 1.65 g of 1-nitro-2,2-bis(methylthio)ethylene in 30 ml. of acetonitrile. The reaction mixture was heated to reflux temperature for about four hours after which time the solvent was removed by evaporation. The resulting residue was chromatographed over silica using ethyl acetate as the eluant. Fractions shown by TLC to contain the desired product were combined and the solvent evaporated therefrom to yield 1-(2-[2-(dimethylaminomethyl)-4-thiazolylmethylthio]ethylamino)-1-methylthio-2-nitroethylene (2.39 g.) Melting at about 68.5—70.0°C. after recrystallization from ethyl acetate.

| | | | | |
|---|---|---|---|---|
| Analysis: Calculated: | C, 41.36; | H, 5.78; | N, 16.08; | S, 27.60; |
| Found: | C, 41.54; | H, 5.61; | N, 16.14; | S, 27.54; |

A reaction mixture, prepared from 1.25 g of 1-[2-([2-(dimethylaminomethyl)-4-thiazolyl]methylthio)-ethylamino]-1-methylthio-2-nitroethylene, 0.27 g of 2-methoxyethylamine and 20 ml of ethanol, was heated to refluxing temperature for about three hours. The solvent was then removed by evaporation and the resulting residue was chromatographed over silica using a gradient elution technique. The desired compound was eluted with a 5:95 methanol:ethyl acetate solvent mixture. Removal of the solvent yielded N-2-([2-(dimethylaminomethyl)-4-thiazolyl]methylthio)ethyl-N'-(2-methoxyethyl) 2-nitro-1,1-ethene-diamine as an oil.

| | | | | |
|---|---|---|---|---|
| Analysis: Calculated: | C, 44.78; | H, 6.71; | N, 18.65; | O, 12.78; |
| Found: | C, 44.65; | H, 6.59; | N, 18.32; | O, 13.03. |

NMR ($CDCl_3$) δ at 2.36 (s, 6H); 2.76 (trip, 2H); 3.40 (s, 3H); 3.1—3.65 (multiplets, 6H); 3.75 (s, 2H); 3.83 (s, 2H); 6.54 (s, 1H); 6.75 (br, 1H); 7.08 (s, 1H); 10.35 (br, 1H)

Following the above procedure, 1-(2-[2-(dimethylaminomethyl)-4-thiazolyl]methylthio)ethylamino]-1-methylthio-2-nitroethylene was reacted with a series of amines: 3-dimethylaminopropylamine, cyclopropylamine, 2-hydroxyethylamine, dimethylamine and ammonia to yield the following products:

N-2-([2-(dimethylaminomethyl)-4-thiazolyl]methylthio)ethyl-N'-(3-dimethylaminopropyl)-2-nitro-1,1-ethenediamine (a glass).

| | | | |
|---|---|---|---|
| Analysis: Calculated: | C, 47.74; | H, 7.51; | N, 20.88 |
| Found: | C, 47.82; | H, 7.24; | N, 21.16 |

NMR ($CDCl_3$): δ at 1.77 (quintet, 2H); 2.23 (s, 3H); 2.28 (s, 3H); 2.36 (s, 6H); 2.23—2.28 (underlying, 2H); 2.72 (trip, 2H); 3.0—3.5 (multiplets, 4H); 3.75 (s, 2H); 3.84 (s, 2H); 6.50 and 6.53 (s, total 1H); 7.11 (s, 1H); 8.6 (br, 1H); 10.2 (br, 1H).

N-2-([2-(dimethylaminomethyl)-4-thiazolyl]methylthio)ethyl-N'-cyclopropyl 2-nitro-1,1-ethenediamine. Melting at 128.5—131.5°C after recrystallization from a methanol/ethyl acetate solvent mixture.

| | | | |
|---|---|---|---|
| Analysis: Calculated: | C, 47.04; | H, 6.49; | N, 19.59 |
| Found: | C, 47.31; | H, 6.12; | N, 19.35 |

N-2-[[2-(dimethylaminomethyl)-4-thiazolyl]methylthio)ethyl-N'-(2-hydroxyethyl) 2-nitro-1,1-ethenediamine obtained as a glass, was eluted from a silica chromatographic column with a 94:5:1 ethyl acetate:methanol:ammonium hydroxide (0.88 sp. gr.) solvent mixture as the eluant. $R_f = 0.43$ on silica using a 95:5 ethanol:ammonium hydroxide solvent mixture.

($CDCl_3$): δ at 2.37 (s, 6H); 2.80 (trip, 2H); 3.1—3.6 (multiplets, 4H); 3.74 (s, 2H); 3.85 (s, 1H); *ca.* 3.8 (multiplet unresolved, 2H); 6.58 (s, 1H); 7.11 (s, 1H); 7.80 (br, 1H); 10.36 (br, 1H)

N-2-([2-(dimethylaminomethyl)-4-thiazolyl]methylthio)ethyl-N',N'-dimethyl-2-nitro-1,1-ethenediamine. The compound was purified by chromatography over silica using a 95:5 ethyl acetate:methanol solvent mixture as the eluent. The compound was an oil.

| | | | | |
|---|---|---|---|---|
| Analysis: Calculated: | C, 45.19; | H, 6.71; | N, 20.27 |
| Found: | C, 45.32; | H, 6.71; | N, 20.54 |

($CDCl_3$): δ at 2.36 (s, 6H); 2.79 (trip, 2H); 2.91 (s, 6H); 3.44 (db. of trip, 2H); 3.74 (s, 2H); 3.86 (s, 2H); 6.48 (s, 1H); 7.12 (s, 1H); 9.46 (br, 1H)

N-([2-(dimethylaminomethyl)-4-thiazolyl]methylthio)ethyl 2-nitro-1,1-ethenediamine melting at 125—7°C after purification by chromatography over silica using a 94:5:1 ethyl acetate-methanol-$NH_4OH$ (0.88 sp. gr.) solvent mixture as the eluant.

| | | | |
|---|---|---|---|
| Analysis: Calculated: | C, 41.62; | H, 6.03; | N, 22.06 |
| Found: | C, 41.38; | H, 6.21; | N, 21.87 |

## Example 18

Preparation of N-2-([2-(dimethylaminomethyl)-4-thiazolyl]methylthio)ethyl-N′-cyanoguanidine.

A reaction mixture was prepared from 1.16 g of 2-([2-(dimethylaminomethyl)-4-thiazolyl]methylthio)-ethylamine, 0.45 g of sodium dicyanamide, 4.8 ml of 1N aqueous hydrochloric acid and 8 ml of n-butanol. The reaction mixture was heated to reflux temperature for about 16 hours and then was filtered. Evaporation of the filtrate to dryness yielded a residue which was purified by chromatography over silica using a 98:2 ethyl acetate:methanol solvent mixture as the eluant. Fractions shown by TLC to contain the desired product were combined and the solvent evaporated therefrom. N-2-([2-(dimethylaminomethyl)-4-thiazolyl]methylthio)ethyl-N′-cyanoguanidine thus obtained was a glass.

| | | | |
|---|---|---|---|
| Analysis: Calculated: | C, 44.27; | H, 6.08; | N, 28.16; |
| Found: | C, 43.91; | H, 5.90; | N, 27.93 |

($CDCl_3$): δ at 2.32 (s, 6H); 2.76 (trip, 2H); 3.50 (db of trip, 2H); 3.74 (s, 2H); 3.83 (s, 2H); 6.38 (br, 2H); 7.01 (s, 1H); 7.64 (br, 1H)

## Example 19

Preparation of N-3-([2-(dimethylaminomethyl)-4-thiazolyl]methylthio)propyl-N′-methyl 2-nitro-1,1-ethene diamine.

Following the procedure of Example 3, 10 g of 2-(dimethylaminomethyl)-4-thiazolylmethanol, 9.2 g of homocysteamine (3-aminopropanethiol) hydrobromide and 100 ml of 48% aqueous hydrobromic acid were heated to reflux temperature for about six hours. Volatile constituents were removed by evaporation and the crystalline residue was triturated with isopropanol. The isopropanol was decanted. This procedure was repeated several times. The crystalline product was finally filtered to yield 7.0 g of 3-([2-(dimethylaminomethyl)-4-thiazolyl]methylthio)propylamine trihydrobromide melting at 179—181°C (hygroscopic).

| | | | | |
|---|---|---|---|---|
| Analysis: Calculated: | C, 24.61; | H, 4.54; | Br, 49.11; | N, 8.61; |
| Found: | C, 24.46; | H, 4.34; | Br, 49.31; | N, 8.38 |

A reaction mixture was prepared from .8 g of the above propylamine and 0.53 g of 1-methylamino-1-methylthio-2-nitroethylene in 10 ml of ethanol. The reaction mixture was heated to reflux temperature for about 20 hours after which time the solvent was removed by evaporation and the resulting residue triturated with ether. N-3-([2-(dimethylaminomethyl)-4-thiazolyl]methylthio)propyl-N′-methyl 2-nitro-1,1-ethenediamine thus prepared had the following physical characteristics:

| | | | |
|---|---|---|---|
| Analysis: Calculated: | C, 45.19; | H, 6.71; | N, 20.27; |
| Found: | C, 45.25; | H, 6.51; | N, 19.99. |

## Example 20

Preparation of 3-[2-([2-(dimethylaminomethyl)-4-thiazolyl]methylthio)ethyl]amino-4-methylamino-3-cyclobutene-1,2-dione.

A solution was prepared from 568 mg of 3,4-dimethoxy-3-cyclobutene-1,2-dione and 15 ml of methanol. A second solution containing 925 mg of 2-([2-(dimethylaminomethyl)-4-thiazolyl]methylthio)-ethylamine in 25 ml of methanol was added to the first solution with stirring over a period of about 1.5 hours. After three hours a small amount of solid was removed from the reaction mixture by filtration. The filtrate was evaporated to a volume of about 10 ml and 6 ml of a 35% solution of methylamine in ethanol was added thereto. After a reaction time of about 2.25 hours, a solid had separated. This solid was filtered and the filter cake washed with methanol. Recrystallization from ethanol gave 0.72 g of 3-[2-([2-(dimethylaminomethyl)-4-thiazolyl]methylthio)ethyl]amino-4-methylamino-3-cyclobutene-1,2-dione melting at 163—167°C.

| | | | | |
|---|---|---|---|---|
| Analysis: Calculated: | C, 49.39; | H, 5.92; | N, 16.46; | O, 9.40. |
| Found: | C, 49.49; | H, 6.01; | N, 16.18; | O, 9.59. |

The compounds of formula (I) in which B is $NRR^6$ are potent $H_2$ receptor antagonists and thus anti-ulcer agents. The relation of the $H_2$ receptors to mammalian gastric secretion is described in an article by Black et al. *Nature, 236,* 385 (1972).

The following assay for $H_2$ receptor blocking activity was employed. Female albino rats were treated with estrone 24 hours prior to the initiation of the experiment. The rats were sacrificed and the uterine horns removed therefrom and suspended at ambient temperatures in isolated organ baths containing De Jalon's solution. After equilibration, the uterine strips are exposed to 50 millimole aqueous potassium chloride, which produces a sustained contraction. When the uterus is so contracted, histamine produces a dose-dependent $H_2$ receptor-mediated relaxation. A control dose-response curve to histamine is constructed on each tissue. Following thorough washout of the histamine after obtaining the control dose-response curve, each antagonist (the compounds of this invention) is added for 30 minutes at a concentration of $10^{-5}$ molar. The uterine strips are then contracted with aqueous potassium chloride in the presence of the antagonist and a second dose-response curve to histamine obtained. In the presence of a competitive antagonist, the dose-response curve to histamine is shifted in parallel to the right with no depression of the maximum relative to the control curve. The dose ratio (DR) is calculated for each concentration of antagonist by dividing the $ED_{50}$ of histamine in the presence of the competitive antagonist by the control $ED_{50}$ for histamine. The dissociation constant ($K_B$) of the antagonist is calculated from the dose-ratios by the following equation:

$$K_B = [\text{antagonist}]/(DR—1)$$

Cimetidine is included as an internal standard.

Results of the above assay carried out on N-methyl-N'-2-([2-(dimethylaminomethyl)-4-thiazolyl]-methylthio)ethyl 2-nitro-1,1-ethenediamine indicated that the compound had an approximately 11 times higher affinity for the $H_2$ receptor than cimetidine. N-Methyl-N'-2-([2-(dimethylaminomethyl)-4-thiazolyl]-methylthio)ethyl-N''-cyanoguanidine had an affinity of about 1.5 times greater than cimetidine. The $K_B$ for the former compound in nanomolar conc. was found to be 87 as compared to a $K_B$ for cimetidine of 871 indicating a relative affinity of about 10 to 1 using a Schild plot.

A second assay for $H_2$ receptor blocking activity employs the isolated bullfrog gastric mucosa — see Warrick and Lin, *Communications in Chem. Pathology and Pharmacology, 13,* 149 (1976). The assay is carried out as follows: The gastric mucosa of the bullfrog (*Rana catesbeiana*) is separated from the musculature of the stomach and placed between a pair of Ussing chambers made of lucite. The chambers are filled with frog Ringer solution and acid secretion is stimulated by addition of histamine to the serosal side of the mucosa at a final concentration of $10^{-5}$ M/l. Acid output is automatically titrated to pH 4.5. After steady response to $10^{-5}$ M/l of histamine is established, the antagonist (a compound of this invention) is added to the serosal chamber and the maximal inhibition by each concentration of the $H_2$-antagonist is recorded. From the dose-response curve, the $ED_{50}$ of the drug is calculated. The relative potency of each unknown antagonist is calculated by dividing the $ED_{50}$ for cimetidine by the $ED_{50}$ of the drug in question. N-methyl-N'-2-([2-(dimethylaminomethyl)-4-thiazolyl]methylthio)ethyl 2-nitro-1,1-ethenediamine had a relative potency of 17.78 compared to 1.0 for cimetidine.

An *in vivo* assay for antisecretory action of drugs on acid secretion utilizes gastric fistula dogs with vagally innervated gastric fistula and vagally dénervated Heidenthain pouch. In this procedure, a steady-state gastric secretion is produced by the iv infusion of histamine. The antisecretory drugs under test are given either intravenously by infusion over a 30 minute period or orally 75 min. prior to collection of gastric secretion from the fistula. N-methyl-N'-2-([2-(dimethylaminomethyl)-4-thiazolyl]methylthio)ethyl 2-nitro-1,1-ethenediamine was about 6.5 times as active as cimetidine by the intravenous route and about 11.0 times as active orally using this procedure.

These last results indicate that N-methyl-N'-2-([2-(dimethylaminomethyl)-4-thiazolyl]methylthio)ethyl 2-nitro-1,1-ethenediamine and other compounds of this invention are better absorbed orally than cimetidine or other recently developed histamine $H_2$ antagonists. This greater oral absorption is also indicated by a relatively greater oral toxicity (compared to *iv* toxicity) for the compounds of this invention. $LD_{50}$'s have been determined for the above ethenediamine and for cimetidine as follows: For N-methyl-N'-2-([2-(dimethylaminomethyl)-4-thiazolyl]methylthio)ethyl 2-nitro-1,1-ethenediamine, the following $LD_{50}$'s were obtained: mouse *iv* 265 mg/kg, mouse oral 1685 mg/kg; rat *iv* above 300 mg/kg, rat oral 1680 mg/kg. Literature $LD_{50}$'s for cimetidine are 150, 2600, 106 and 5000 mg/kg respectively. The relative lack (relative to cimetidine) of toxicity by the intravenous route of the compounds of this invention is surprising as is the greater oral absorption.

The above figures as to potency and toxicity indicate a favorable therapeutic ratio for the compounds of this invention. Preliminary tests also indicate that the compounds of this invention have a longer duration of action than cimetidine.

In utilizing the compounds of this invention as antisecretory agents, either the parenteral or oral route of administration may be employed.

In one embodiment of the invention, there is provided a pharmaceutical formulation which comprises as an active ingredient, a compound of formula (I) in which B is $NRR^6$, or a pharmaceutically-acceptable salt thereof, associated with one or more pharmaceutically-acceptable carriers therefor. Orally-acceptable

formulations such as capsules or tablets constitute the preferred mode of administration.

For oral dosage, a suitable quantity of a free base of this invention, or a pharmaceutically-acceptable salt thereof, is mixed with one or more conventional excipients such as starch and the mixture placed in telescoping gelatin capsules or compressed into tablets each typically containing from 100—400 mg of active ingredients. The tablets may be scored if lower or divided dosages are to be used. For parenteral administration via an *iv* infusion, an isotonic solution of a salt is preferably employed although a soluble free base is also useful in isotonic preparations.

Because of the higher oral absorption and longer duration of action of the compounds of this invention, particularly N-methyl-N'-2-([2-(dimethylaminomethyl)-4-thiazolyl]methylthio)ethyl 2-nitro-1,1-ethenediamine, it is believed that oral administration of about 50—80 mg three to four times a day will suffice to control acid secretion in ulcer patients and thus alleviate ulcer symptoms. Generally, however, the compounds of this invention are administered to humans orally in a daily dosage range of 140—800 mg. Smaller dosages at more frequent intervals may also be employed. The preferred oral dosage range is about 2—5 mg/kg/day of mammalian body weight, although a dosage range of from 1—10 mg/kg/day can be used.

**Claims for the Contracting States: BE CH DE FR GB IT LI LU NL SE**

1. A thiazole derivative of formula (I):

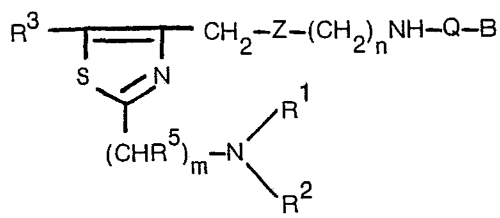

(I)

wherein

each of $R^1$ and $R^2$ independently represent hydrogen, $C_{1-4}$ alkyl, benzyl or benzoyl; or taken together with the adjacent nitrogen atom, form a saturated heterocyclic ring containing from 5 to 7 ring atoms;

$R^3$ is hydrogen or $C_{1-4}$ alkyl;

Z is O, S, or $CH_2$;

n is 2 or 3 when Z is O or S and n is 1, 2 or 3 when Z is $CH_2$;

$R^5$ is hydrogen or $C_{1-4}$ alkyl;

m is 1, 2 or 3;

Q is 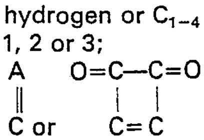 or ;

wherein

A is N—CN, N—$NO_2$, CH—$NO_2$, S, O, NH, N—$SO_2$-aryl, N—$SO_2$—$C_{1-4}$ alkyl, N—CO—$NH_2$, N—CO—$C_{1-4}$ alkyl, N—$CO_2$—$C_{1-4}$ alkyl; CH—$SO_2$-aryl or CH—$SO_2$—$C_{1-4}$ alkyl, wherein aryl is phenyl, halophenyl, $C_{1-4}$ alkylphenyl or $C_{1-4}$ alkoxyphenyl; and

B is $NRR^6$, wherein R and $R^6$ are independently hydrogen, $C_{1-5}$ alkyl, $C_{3-6}$ cycloalkylmethyl, hydroxy $C_{2-5}$ alkyl, $C_{3-6}$ cycloalkyl, alkoxyalkyl or dialkylaminoalkyl wherein the total number of carbons is less than 8 and there is at least a two carbon chain between the hetero atoms; or $YR^4$, wherein Y is oxygen or sulfur and $R^4$ is $C_{1-5}$ alkyl, —$CH_2$ $C_{2-4}$ alkenyl or benzyl;

or a pharmaceutically-acceptable salt of a compound in which B is $NRR^6$.

2. A thiazole derivative as claimed in claim 1 wherein $R^1$ and $R^2$ individually represent hydrogen or $C_{1-3}$ alkyl, one only of $R^1$ and $R^2$ may represent benzyl or benzoyl, or $R^1$ and $R^2$ taken together with the adjacent nitrogen atom represent a piperidino, pyrrolidino or morpholino group, provided that only one of $R^1$ and $R^2$ can be hydrogen when Z is $CH_2$; $R^3$ is hydrogen or $C_{1-3}$ alkyl; $R^4$ is $C_{1-5}$ alkyl; $R^5$ is hydrogen or methyl; A is N—CN, N—$NO_2$, CH—$NO_2$, S, O, NH, N—$SO_2$-aryl, N—$SO_2$—$C_{1-3}$ alkyl, N—CO—$NH_2$, N—CO—$C_{1-3}$ alkyl, N—$CO_2$—$C_{1-3}$ alkyl, CH—$SO_2$-aryl or CH—$SO_2$—$CH_3$, where aryl is phenyl, halophenyl, $C_{1-3}$ alkylphenyl or $C_{1-3}$ alkoxyphenyl, $R^6$ is hydrogen; provided that when B is $YR^4$, Q is C=A;

or a pharmaceutically-acceptable acid-addition salt of a compound in which B is $NRR^6$.

3. A thiazole derivative as claimed in claim 1 or 2, wherein Z is sulfur.

4. A thiazole derivative as claimed in any one of claims 1 to 3, wherein n is 2.

5. A thiazole derivative as claimed in any one of the preceding claims, wherein $R^3$ is hydrogen.

6. A thiazole derivative as claimed in any one of the preceding claims, wherein $R^5$ is hydrogen.

7. A thiazole derivative as claimed in any one of the preceding claims, wherein m is 1.

8. A thiazole derivative as claimed in any one of the preceding claims, wherein $R^1$ and $R^2$ are methyl.

9. A thiazole derivative as claimed in claim 8, wherein the —$(CHR^5)_mNR^1R^2$ group is dimethylaminomethyl.

10. A thiazole derivative as claimed in any one of the preceding claims, wherein A is NCN or $CHNO_2$.

11. A thiazole derivative as claimed in any one of the preceding claims wherein B is $NRR^6$ where R is hydrogen and $R^6$ is $C_{1-4}$ alkyl.

12. A thiazole derivative as claimed in claim 11, wherein B is methylamino.

13. N-Methyl-N'-2-([2-(dimethylaminomethyl)-4-thiazolyl]methylthio)ethyl-N"-cyanoguanidine, or a pharmaceutically-acceptable salt thereof.

14. N-Methyl-N'-([2-(dimethylaminomethyl)-4-thiazolyl]methylthio)ethyl-2-nitro-1,1-ethenediamine, or a pharmaceutically-acceptable salt thereof.

15. A pharmaceutical formulation which comprises as an active ingredient, a thiazole derivative, or a pharmaceutically-acceptable salt thereof, as claimed in any one of claims 1 to 14, associated with one or more pharmaceutically-acceptable carriers therefor.

16. A pharmaceutical formulation as claimed in claim 15 in the form of a capsule or tablet.

17. A process for preparing a thiazole derivative of formula (I) as claimed in any one of claims 1 to 14 which comprises:

(a) reacting an amine of formula:

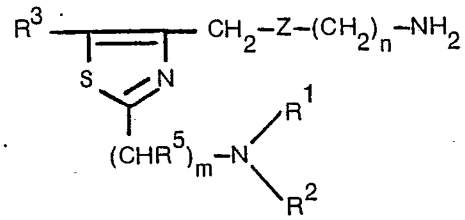

with

(i) a compound of formula $L^1QB$ wherein $L^1$ is a leaving group; or

(ii) a compound of formula $R^6$—N=C=A to form a thiazole of formula (I) in which Q is —C=A and R is hydrogen.

(b) reacting a compound of formula:

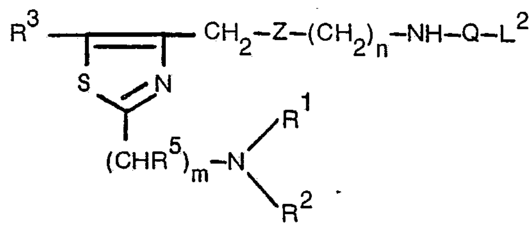

where $L^2$ is a leaving group, with an amine of formula $HNRR^6$;

(c) reacting a compound of formula:

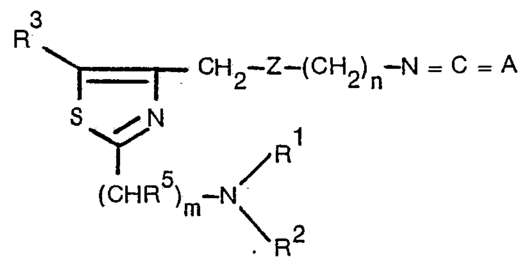

with an amine of formula $R^6NH_2$, to form a thiazole of formula (I) in which Q is C=A and B is $NHR^6$; or

(d) fusing a compound of formula:

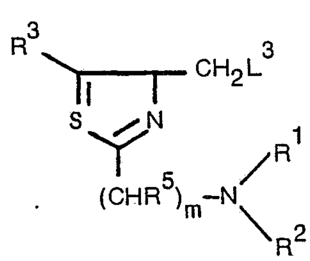

where $L^3$ is a good leaving group, with a thiol of formula

$HS(CH_2)_n—NH—CA—NRR^6$.

to form a thiazole of formula (I) in which Z is sulfur, Q is C=A and B is $NRR^6$.

18. A thiazole derivative of formula (I), or a pharmaceutically-acceptable salt thereof, as claimed in any one of claims 1 to 14, for use in inhibiting gastric acid secretion.

**Claims for the Contracting State: AT**

1. A process for preparing a thiazole derivative of formula (I):

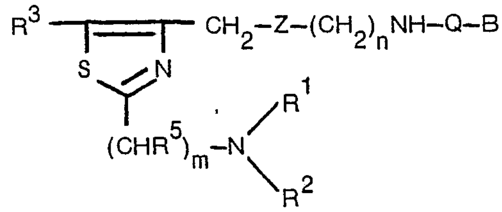

(I)

wherein

each of $R^1$ and $R^2$ independently represent hydrogen, $C_{1-4}$ alkyl, benzyl or benzoyl; or taken together with the adjacent nitrogen atom, form a saturated heterocyclic ring containing from 5 to 7 ring atoms;

$R^3$ is hydrogen or $C_{1-4}$ alkyl;

Z is O, S, or $CH_2$;

n is 2 or 3 when Z is O or S and n is 1, 2 or 3 when Z is $CH_2$;

$R^5$ is hydrogen or $C_{1-4}$ alkyl;

m is 1, 2 or 3;

Q is 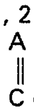 or 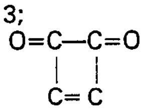 ;

wherein

A is N—CN, N—$NO_2$, CH—$NO_2$, S, O, NH, N—$SO_2$-aryl, N—$SO_2$—$C_{1-4}$ alkyl, N—CO—$NH_2$, N—CO—$C_{1-4}$ alkyl, N—$CO_2$—$C_{1-4}$ alkyl; CH—$SO_2$-aryl or CH—$SO_2$—$C_{1-4}$ alkyl, wherein aryl is phenyl, halophenyl, $C_{1-4}$ alkylphenyl or $C_{1-4}$ alkoxyphenyl; and

B is $NRR^6$, wherein R and $R^6$ are independently hydrogen, $C_{1-5}$ alkyl, $C_{3-6}$ cycloalkylmethyl, hydroxy $C_{2-5}$ alkyl, $C_{3-6}$ cycloalkyl, alkoxyalkyl or dialkylaminoalkyl wherein the total number of carbons is less than 8 and there is at least a two carbon chain between the hetero atoms; or a pharmaceutically-acceptable salt thereof, which process comprises:

(a) reacting an amine of formula:

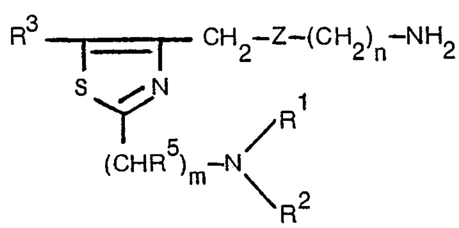

with

(i) a compound of formula $L^1QB$ wherein $L^1$ is a leaving group; or

(ii) a compound of formula $R^6$—N=C=A to form a thiazole of formula (I) in which Q is —C=A and R is hydrogen.

(b) reacting a compound of formula:

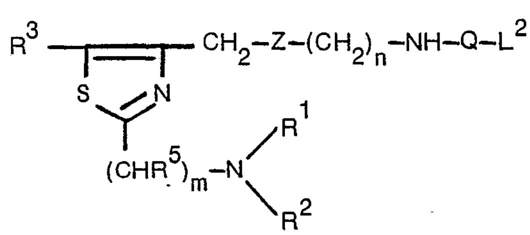

where $L^2$ is a leaving group, with an amine of formula $HNRR^6$;

(c) reacting a compound of formula:

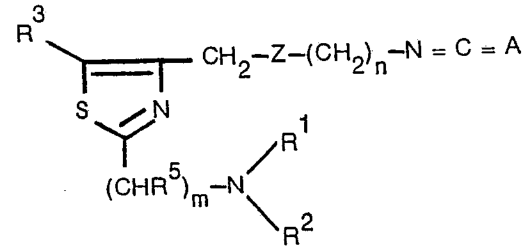

with an amine of formula $R^6NH_2$, to form a thiazole of formula (I) in which Q is C=A; or

(d) fusing a compound of formula:

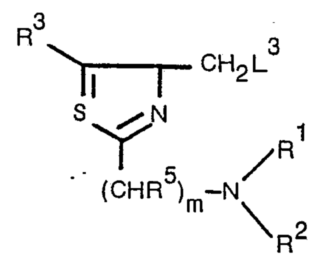

where $L^3$ is a good leaving group, with a thiol of formula

$$HS(CH_2)_n—NH—CA—NRR^6.$$

to form a thiazole of formula (I) in which Z is sulfur, and Q is C=A.

2. A process according to claim 1 for preparing a thiazole derivative of formula (I) wherein $R^1$ and $R^2$ individually represent hydrogen or $C_{1-3}$ alkyl, one only of $R^1$ and $R^2$ may represent benzyl or benzoyl, or $R^1$ and $R^2$ taken together with the adjacent nitrogen atom represent a piperidino, pyrrolidino or morpholino group, provided that only one of $R^1$ and $R^2$ can be hydrogen when Z is $CH_2$; $R^3$ is hydrogen or $C_{1-3}$ alkyl; $R^4$ is $C_{1-5}$ alkyl; $R^5$ is hydrogen or methyl; A is N—CN, N—$NO_2$, CH—$NO_2$, S, O, NH, N—$SO_2$-aryl, N—$SO_2$—$C_{1-3}$ alkyl, N—CO—$NH_2$, N—CO—$C_{1-3}$ alkyl, N—$CO_2$—$C_{1-3}$ alkyl, CH—$SO_2$-aryl or CH—$SO_2$—$CH_3$, where aryl is phenyl, halophenyl, $C_{1-3}$ alkylphenyl or $C_{1-3}$ alkoxyphenyl, $R^6$ is hydrogen; and Q is C=A; or a pharmaceutically-acceptable acid-addition salt thereof.

3. A process according to claim 1 for preparing N-methyl-N'-2-([2-(dimethylaminomethyl)-4-thiazolyl]methylthio)ethyl-N''-cyanoguanidine, or a pharmaceutically-acceptable salt thereof.

4. A process according to claim 1 for preparing N-methyl-N'-([2-(dimethylaminomethyl)-4-thiazolyl]methylthio)ethyl-2-nitro-1,1-ethenediamine, or a pharmaceutically-acceptable salt thereof.

5. A thiazole of formula (I), or a pharmaceutically-acceptable salt, thereof, whenever prepared by a process according to any one of claims 1 to 4.

**Revendications pour les Etats contractants: BE CH DE FR GB IT LI LU NL SE**

1. Dérivé de thiazole de formule (I):

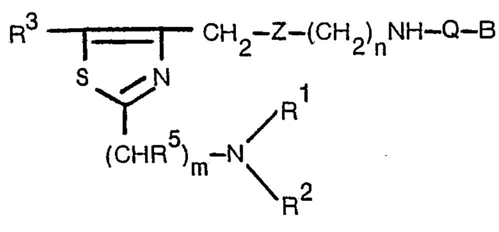

(I)

dans laquelle

$R^1$ et $R^2$ représentent chacun indépendamment l'un de l'autre un atome d'hydrogène, un groupe alkyle en $C_1$—$C_4$, un groupe benzyle ou un groupe benzoyle; ou, pris ensemble avec l'atome d'azote adjacent, ils forment un noyau hétérocyclique saturé contenant 5 à 7 atomes cycliques;

$R^3$ représente un atome d'hydrogène ou un groupe alkyle en $C_1$—$C_4$;

Z représente O, S ou $CH_2$;

n est égal à 2 ou 3 lorsque Z représente O ou S, tandis qu'il est égal à 1, 2 ou 3 lorsque Z représente $CH_2$;

$R^5$ représente un atome d'hydrogène ou un groupe alkyle en $C_1$—$C_4$;

m est égal à 1, 2 ou 3;

Q représente 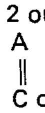 ou 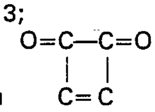 ;

où

A représente N—CN, N—$NO_2$, CH—$NO_2$, S, O, NH, N—$SO_2$-aryle, N—$SO_2$-alkyle en $C_1$—$C_4$, N—CO—$NH_2$, N—CO-alkyle en $C_1$—$C_4$, N—$CO_2$-alkyle en $C_1$—$C_4$; CH—$SO_2$-aryle ou CH—$SO_2$-alkyle en $C_1$—$C_4$ où "aryle" représente un groupe phényle, un groupe halophényle, un groupe alkyl(en $C_1$—$C_4$)phényle ou un groupe alcoxy(en $C_1$—$C_4$)phényle; et

B représente $NRR^6$ où R et $R^6$ représentent chacun indépendamment l'un de l'autre un atome d'hydrogène, un groupe alkyle en $C_1$—$C_5$, un groupe cycloalkyl(en $C_3$—$C_6$)méthyle, un groupe hydroxyalkyle en $C_2$—$C_5$, un groupe cycloalkyle en $C_3$—$C_6$, un groupe alcoxyalkyle ou un groupe dialkylaminoalkyle dans lequel le nombre total d'atomes de carbone est inférieur à 8, tandis qu'il y a au moins une chaîne à 2 atomes de carbone entre les hétéro-atomes; ou $YR^4$ où Y représente un atome d'oxygène ou de soufre et $R^4$ représente un groupe alkyle en $C_1$—$C_5$, un groupe —$CH_2$-alcényle en $C_2$—$C_4$ ou un groupe benzyle;

ou encore un sel pharmaceutiquement acceptable d'un composé dans lequel B représente $NRR^6$.

2. Dérivé de thiazole selon la revendication 1, dans lequel $R^1$ et $R^2$ représentent chacun individuellement un atome d'hydrogène ou un groupe alkyle en $C_1$—$C_3$, un seul des radicaux $R^1$ et $R^2$ peut représenter un groupe benzyle ou un groupe benzoyle, ou encore $R^1$ et $R^2$, pris ensemble avec l'atome d'azote adjacent, représentent un groupe pipéridino, un groupe pyrrolidino ou un groupe morpholino, avec cette réserve qu'un seul des radicaux $R^1$ et $R^2$ peut être un atome d'hydrogène lorsque Z représente $CH_2$; $R^3$ représente un atome d'hydrogène ou un groupe alkyle en $C_1$—$C_3$; $R^4$ représente un groupe alkyle en $C_1$—$C_5$; $R^5$ représente un atome d'hydrogène ou un groupe méthyle; A représente N—CN, N—$NO_2$, CH—$NO_2$, S, O, NH, N—$SO_2$-aryle, N—$SO_2$-alkyle en $C_1$—$C_3$, N—CO—$NH_2$, N—CO-alkyle en $C_1$—$C_3$, N—$CO_2$-alkyle en $C_1$—$C_3$, CH—$SO_2$-aryle ou CH—$SO_2$—$CH_3$, où "aryle" représente un groupe phényle, un groupe halophényle, un groupe alkyl(en $C_1$—$C_3$)phényle ou un groupe alcoxy(en $C_1$—$C_3$)phényle, $R^6$ représente un atome d'hydrogène, avec cette réserve que, lorsque B représente $YR^4$, Q représente C=A;

ou un sel d'addition d'acide pharmaceutiquement acceptable d'un composé dans lequel B représente $NRR^6$.

3. Dérivé de thiazole selon la revendication 1 ou 2, dans lequel Z représente un atome de soufre.

4. Dérivé de thiazole selon l'une quelconque des revendications 1 à 3, dans lequel n est égal à 2.

5. Dérivé de thiazole selon l'une quelconque des revendications précédentes, dans lequel $R^3$ représente un atome d'hydrogène.

6. Dérivé de thiazole selon l'une quelconque des revendications précédentes, dans lequel $R^5$ représente un atome d'hydrogène.

7. Dérivé de thiazole selon l'une quelconque des revendications précédentes, dans lequel m est égal à 1.

8. Dérivé de thiazole selon l'une quelconque des revendications précédentes, dans lequel $R^1$ et $R^2$ représentent chacun un groupe méthyle.

9. Dérivé de thiazole selon la revendication 8, dans lequel le groupe —$(CHR^5)_mNR^1R^2$ est le groupe diméthylaminométhyle.

10. Dérivé de thiazole selon l'une quelconque des revendications précédentes, dans lequel A représente NCN ou $CHNO_2$.

11. Dérivé de thiazole selon l'une quelconque des revendications précédentes, dans lequel B représente $NRR^6$ où R est un atome d'hydrogène et $R^6$ est un groupe alkyle en $C_1$—$C_4$.

12. Dérivé de thiazole selon la revendication 11, dans lequel B est le groupe méthylamino.

13. La N-méthyl-N'-2-([2-(diméthylaminométhyl)-4-thiazolyl]méthylthio)éthyl-N"-cyanoguanidine ou un de ses sels pharmaceutiquement acceptables.

14. La N-méthyl-N'-([2-(diméthylaminométhyl)-4-thiazolyl]méthylthio)éthyl-2-nitro-1,1-éthène-diamine ou un de ses sels pharmaceutiquement acceptables.

15. Formulation pharmaceutique comprenant, comme ingrédient actif, un dérivé de thiazole ou un de ses sels pharmaceutiquement acceptables selon l'une quelconque des revendications 1 à 14, en association avec un ou plusieurs supports pharmaceutiquement acceptables pour ce dérivé ou ce sel.

16. Formulation pharmaceutique selon la revendication 15, sous forme d'une capsule ou d'un comprimé.

17. Procédé de préparation d'un dérivé de thiazole répondant à la formule (I) selon l'une quelconque des revendications 1 à 14, caractérisé en ce qu'il comprend les étapes qui consistent à:

(a) faire réagir une amine de formule:

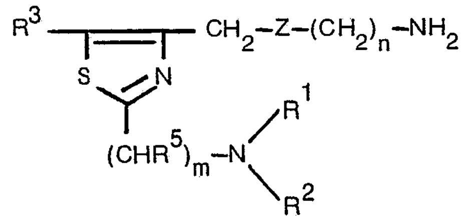

avec

(i) un composé de formule $L^1QB$ dans laquelle $L^1$ représente un groupe qui s'éloigne; ou

(ii) un composé de formule $R^6$—N=C=A pour former un thiazole de formule (I) dans laquelle Q représente —C=A et R représente un atome d'hydrogène;

(b) faire réagir un composé de formule:

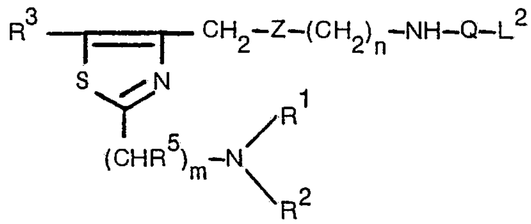

dans laquelle $L^2$ représente un groupe qui s'éloigne, avec une amine de formule $HNRR^6$;

(c) faire réagir un composé de formule:

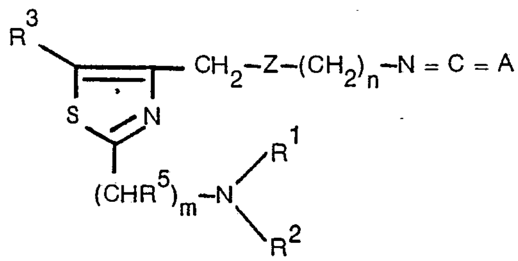

avec une amine de formule $R^6NH_2$ pour former un thiazole de formule (I) dans laquelle Q représente C=A et B représente $NHR^6$; ou

(d) fusionner un composé de formule:

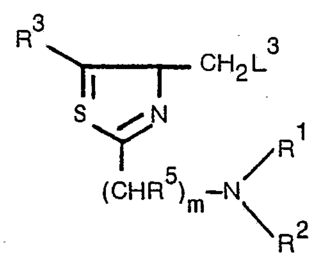

dans laquelle $L^3$ est un bon groupe qui s'éloigne, avec un thiol de formule:

$$HS(CH_2)_n—NH—CA—NRR^6$$

pour former un thiazole de formule (I) dans laquelle Z représente un atome de soufre, Q représente C=A et B représente $NRR^6$.

18. Dérivé de thiazole de formule (I) ou un de ses sels pharmaceutiquement acceptables selon l'une quelconque des revendications 1 à 14, en vue d'inhiber la sécrétion d'acide gastrique.

**Revendications pour l'Etat contractant: AT**

1. Procédé de préparation d'un dérivé de thiazole de formule (I):

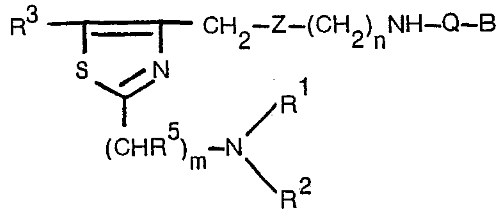

(I)

dans laquelle

$R^1$ et $R^2$ représentent chacun indépendamment l'un de l'autre un atome d'hydrogène, un groupe alkyle en $C_1$—$C_4$, un groupe benzyle ou un groupe benzoyle; ou, pris ensemble avec l'atome d'azote adjacent, ils forment un noyau hétérocyclique saturé contenant 5 à 7 atomes cycliques;

$R^3$ représente un atome d'hydrogène ou un groupe alkyle en $C_1$—$C_4$;

Z représente O, S ou $CH_2$;

n est égal à 2 ou 2 lorsque Z représente O ou S, tandis qu'il est égal à 1, 2 ou 3 lorsque Z représente $CH_2$;

$R^5$ représente un atome d'hydrogène ou un groupe alkyle en $C_1$—$C_4$;

m est égal à 1, 2 ou 3;

A O=C—C=O

‖ | |

Q représente C ou C=C ;

où

A représente N—CN, N—$NO_2$, CH—$NO_2$, S, O, NH, N—$SO_2$-aryle, N—$SO_2$-alkyle en $C_1$—$C_4$, N—CO—$NH_2$, N—CO-alkyle en $C_1$—$C_4$, N—$CO_2$-alkyle en $C_1$—$C_4$, CH—$SO_2$-aryle ou CH—$SO_2$-alkyle en $C_1$—$C_4$ où "aryle" représente un groupe phényle, un groupe halophényle, un groupe alkyl(en $C_1$—$C_4$)phényle ou un groupe alcoxy(en $C_1$—$C_4$)phényle; et

B représente $NRR^6$ où R et $R^6$ représentent chacun indépendamment l'un de l'autre un atome d'hydrogène, un groupe alkyle en $C_1$—$C_5$, un groupe cycloalkyl(en $C_3$—$C_6$)méthyle, un groupe hydroxy-alkyle en $C_2$—$C_5$, un groupe cycloalkyle en $C_3$—$C_6$, un groupe alcoxyalkyle ou un groupe dialkylaminoalkyle dans lequel le nombre total d'atomes de carbone est inférieur à 8, tandis qu'il y a au moins une chaîne à deux atomes de carbone entre les hétéro-atomes; ou d'un sel pharmaceutiquement acceptable de ce dérivé, ce procédé comprenant les étapes consistant à:

(a) faire réagir une amine de formule:

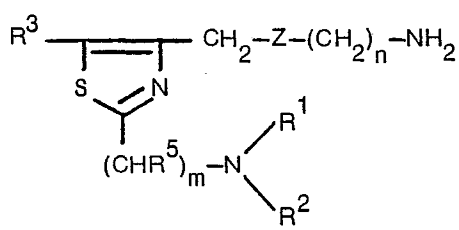

avec

(i) un composé de formule $L^1QB$ dans laquelle $L^1$ représente un groupe qui s'éloigne; ou

(ii) un composé de formule $R^6$—N=C=A pour former un thiazole de formule (I) dans laquelle Q représente —C=A et R représente un atome d'hydrogène;

(b) faire réagir un composé de formule:

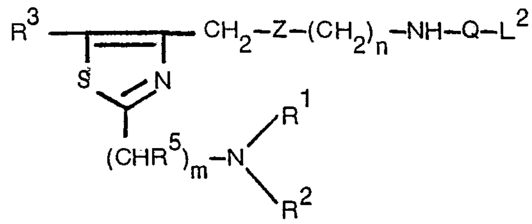

dans laquelle $L^2$ représente un groupe qui s'éloigne, avec une amine de formule $HNRR^6$;

(c) faire réagir un composé de formule:

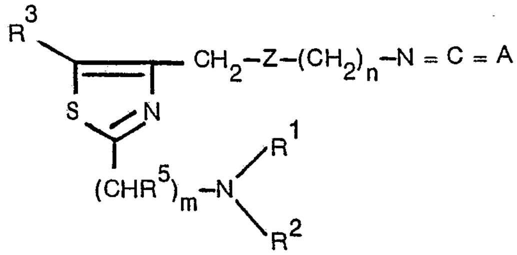

avec une amine de formule $R^6NH_2$ pour former un thiazole de formule (I) dans laquelle Q représente C=A; ou

(d) fusionner un composé de formule:

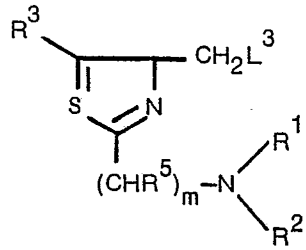

dans laquelle $L^3$ est un bon groupe qui s'éloigne, avec un thiol de formule:

$$HS(CH_2)_n—NH—CA—NRR^6$$

pour former un thiazole de formule (I) dans laquelle Z,représente un atome de soufre et Q représente C=A.

2. Procédé selon la revendication 1 pour préparer un dérivé de thiazole de formule (I) dans laquelle $R^1$ et $R^2$ représentent chacun individuellement un atome d'hydrogène ou un groupe alkyle en $C_1—C_3$, un seul des radicaux $R^1$ et $R^2$ peut représenter un groupe benzyle ou un groupe benzoyle, ou $R^1$ et $R^2$, pris ensemble avec l'atome d'azote adjacent, représentent un groupe pipéridino, un groupe pyrrolidino ou un groupe morpholino, avec cette réserve qu'un seul des radicaux $R^1$ et $R^2$ peut représenter un atome d'hydrogène lorsque Z représente $CH_2$; $R^3$ représente un atome d'hydrogène ou un groupe alkyle en $C_1—C_3$; $R^4$ représente un groupe alkyle en $C_1—C_5$; $R^5$ représente un atome d'hydrogène ou un groupe méthyle; A représente N—CN, N—$NO_2$, CH—$NO_2$, S, O, NH, N—$SO_2$-aryle, N—$SO_2$-alkyle en $C_1—C_3$, N—CO—$NH_2$, N—CO-alkyle en $C_1—C_3$, N—$CO_2$-alkyle en $C_1—C_3$, CH—$SO_2$-aryle ou CH—$SO_2$—$CH_3$, où "aryle" représente un groupe phényle, un groupe halophényle, un groupe alkyl(en $C_1—C_3$)phényle ou un groupe alcoxy(en $C_1—C_3$)phényle, $R^6$ représente un atome d'hydrogène et Q représente C=A;
ou d'un sel d'addition d'acide pharmaceutiquement acceptable de ce dérivé.

3. Procédé selon la revendication 1, pour la préparation de la N-méthyl-N'-2-([2-(diméthylaminométhyl)-4-thiazolyl]méthylthio)éthyl-N"-cyanoguanidine ou d'un de ses sels pharmaceutiquement acceptables.

4. Procédé selon la revendication 1 pour la préparation de la N-méthyl-N'-([2-(diméthylaminométhyl)-4-thiazolyl]méthylthio)éthyl-2-nitro-1,1-éthène-diamine ou d'un de ses sels pharmaceutiquement acceptables.

5. Thiazole de formule (I) ou un de ses sels pharmaceutiquement acceptables, préparé par un procédé selon l'une quelconque des revendications 1 à 4.

**Patentansprüche für die Vertragsstaaten: BE CH DE FR GB IT LI LU NL SE**

1. Thiazolderivate der Formel (I)

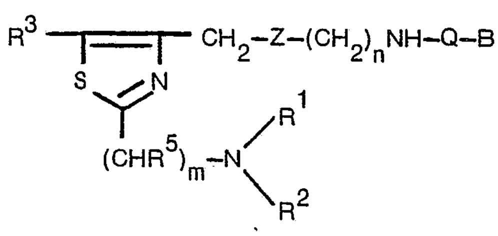

(I)

worin

$R^1$ und $R^2$ unabhängig Wasserstoff, $C_1$—$C_4$-Alkyl, Benzyl oder Benzoyl sind oder zusammen mit dem benachbarten Stickstoffatom einen gesättigten heterocyclischen Ring bilden, der 5 bis 7 Ringatome enthält,

$R^3$ Wasserstoff oder $C_1$—$C_4$-Alkyl ist,

Z für O, S oder $CH_2$ steht,

n für 2 oder 3 steht, falls Z für O oder S steht, und

n für 1, 2 oder 3 steht, falls Z für $CH_2$ steht,

$R^5$ Wasserstoff oder $C_1$—$C_4$-Alkyl ist,

m für 1, 2 oder 3 steht,

Q für 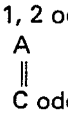 oder 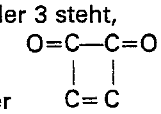 steht,

worin

A für N—CN, N—$NO_2$, CH—$NO_2$, S, O, NH, N—$SO_2$-Aryl, N—$SO_2$—$C_1$—$C_4$-Alkyl, N—CO—$NH_2$, N—CO—$C_1$—$C_4$-Alkyl, N—$CO_2$—$C_1$—$C_4$-Alkyl, CH—$SO_2$-Aryl oder CH—$SO_2$—$C_1$—$C_4$-Alkyl steht, wobei Aryl Phenyl, Halogenphenyl, $C_1$—$C_4$-Alkylphenyl oder $C_1$—$C_4$-Alkoxyphenyl ist, und

B für $NRR^6$ steht, worin R und $R^6$ unabhängig Wasserstoff, $C_1$—$C_5$-Alkyl, $C_3$—$C_6$-Cycloalkylmethyl, Hydroxy-$C_2$—$C_5$-Alkyl, $C_3$—$C_6$-Cycloalkyl, Alkoxyalkyl oder Dialkylaminoalkyl sind, wobei die Gesamtzahl an Kohlenstoffen weniger als 8 beträgt und sich zwischen den Heteroatomen eine Kette aus wenigstens zwei Kohlenstoffatomen befindet, oder

B für $YR^4$ steht, worin Y Sauerstoff oder Schwefel ist und $R^4$ für $C_1$—$C_5$-Alkyl, $CH_2$—$C_2$—$C_4$-Alkenyl oder Benzyl steht,

und die pharmazeutisch annehmbaren Salze der Verbindungen, worin B für $NRR^6$ steht.

2. Thiazolderivat nach Anspruch 1, dadurch gekennzeichnet, daß $R^1$ und $R^2$ unabhängig Wasserstoff oder $C_1$—$C_3$-Alkyl bedeuten, nur einer der Substituenten $R^1$ und $R^2$ Benzyl oder Benzoyl ist oder $R^1$ und $R^2$ zusammen mit dem benachbarten Stickstoffatom eine Piperidino-Pyrrolidino- oder Morpholinogruppe darstellen, mit der Maßgabe, daß nur einer der Substituenten $R^1$ und $R^2$ Wasserstoff sein kann, falls Z für $CH_2$ steht, $R^3$ Wasserstoff oder $C_1$—$C_3$-Alkyl ist, $R^4$ für $C_1$—$C_5$-Alkyl steht, $R^5$ Wasserstoff oder Methyl ist, A für N—CN, N—$NO_2$, CH—$NO_2$, S, O, NH, N—$SO_2$-Aryl, N—$SO_2$—$C_1$—$C_3$-Alkyl, N—CO—$NH_2$, N—CO—$C_1$—$C_3$-Alkyl, N—$CO_2$—$C_1$—$C_3$-Alkyl, CH—$SO_2$-Aryl oder CH—$SO_2$—$CH_3$ steht, worin Aryl Phenyl, Halogenphenyl, $C_1$—$C_3$-Alkylphenyl oder $C_1$—$C_3$-Alkoxyphenyl ist und $R^6$ Wasserstoff ist,.mit der Maßgabe, daß Q für C=A steht, falls B für $YR^4$ steht,

oder ein pharmazeutisch annehmbares Säureadditionssalz einer Verbindung, worin B für $NRR^6$ steht.

3. Thiazolderivat nach Anspruch 1 oder 2, dadurch gekennzeichnet, daß Z Schwefel ist.

4. Thiazolderivat nach einem der Ansprüche 1 bis 3, dadurch gekennzeichnet, daß n für 2 steht.

5. Thiazolderivat nach einem der vorhergehenden Ansprüche, dadurch gekennzeichnet, daß $R^3$ Wasserstoff ist.

6. Thiazolderivat nach einem der vorhergehenden Ansprüche, dadurch gekennzeichnet, daß $R^5$ Wasserstoff ist.

7. Thiazolderivat nach einem der vorhergehenden Ansprüche, dadurch gekennzeichnet, daß m für 1 steht.

8. Thiazolderivat nach einem der vorhergehenden Ansprüche, dadurch gekennzeichnet, daß $R^1$ und $R^2$ Methyl sind.

9. Thiazolderivat nach Anspruch 8, dadurch gekennzeichnet, daß die Gruppe $—(CHR^5)_mNR^1R^2$ Dimethylaminomethyl ist.

10. Thiazolderivat nach einem der vorhergehenden Ansprüche, dadurch gekennzeichnet, daß A für NCN oder $CHNO_2$ steht.

11. Thiazolderivat nach einem der vorhergehenden Ansprüche, dadurch gekennzeichnet, daß B für $NRR^6$ steht, worin R Wasserstoff ist und $R^6$ für $C_1$—$C_4$-Alkyl steht.

12. Thiazolderivat nach Anspruch 11, dadurch gekennzeichnet, daß B Methylamino ist.

13. N-Methyl-N'-2-([2-(dimethylaminomethyl)-4-thiazolyl]methylthio)ethyl-N''-cyanoguanidin oder ein pharmazeutisch annehmbares Salz hiervon.

14. N-Methyl-N'-([2-(dimethylaminomethyl)-4-thiazolyl]methylthio)ethyl-2-nitro-1,1-ethendiamin oder ein pharmazeutisch annehmbares Salz hiervon.

15. Pharmazeutische Formulierung, dadurch gekennzeichnet, daß sie ein Thiazolderivat oder ein pharmazeutisch annehmbares Salz hiervon nach einem der Ansprüche 1 bis 14 als Wirkstoff in Verbindung mit einem oder mehreren pharmazeutisch annehmbaren Trägern hierfür enthält.

16. Pharmazeutische Formulierung nach Anspruch 15, dadurch gekennzeichnet, daß sie die Form einer Kapsel oder einer Tablette hat.

17. Verfahren zur Herstellung eines Thiazolderivats der Formel (I) nach einem der Ansprüche 1 bis 14, dadurch gekennzeichnet, daß man

(a) ein Amin der Formel

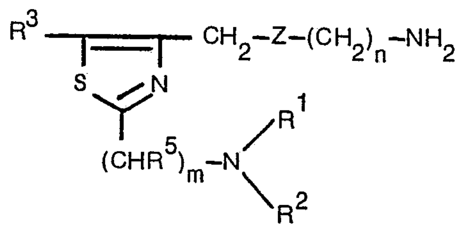

mit

(i) einer Verbindung der Formel $L^1QB$, worin $L^1$ eine Abgangsgruppe ist, oder

(ii) einer Verbindung der Formel $R^6$—N=C=A unter Bildung eines Thiazols der Formel (I), worin Q für —C=A steht und R Wasserstoff ist, umsetzt,

(b) eine Verbindung der Formel

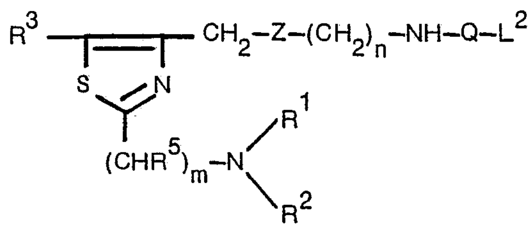

worin $L^2$ eine Abgangsgruppe ist, mit einem Amin der Formel $HNRR^6$ umsetzt,

(c) eine Verbindung der Formel

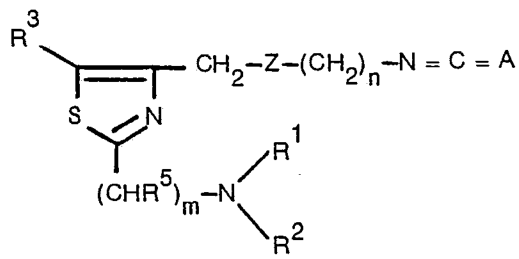

mit einem Amin der Formel $R^6NH_2$ unter Bildung eines Thiazols der Formel (I), worin Q für C=A steht und B für $NHR^6$ steht, umsetzt oder

(d) eine Verbindung der Formel

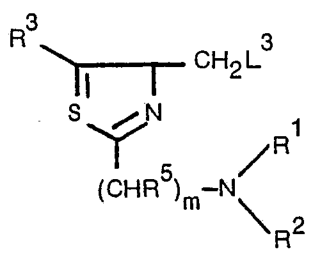

worin $L^3$ eine gute Abgangsgruppe ist, mit einem Thiol der Formel

$$HS(CH_2)_n\text{—NH—CA—}NRR^6$$

unter Bildung eines Thiazols der Formel (I), worin Z Schwefel ist und Q für C=A steht und B für $NRR^6$ steht, verschmilzt.

18. Verwendung eines Thiazolderivats der Formel (I) oder eines pharmazeutisch annehmbaren Salzes hiervon nach einem der Ansprüche 1 bis 14 zur Hemmung der Magensäuresekretion.

**Patentansprüche für den Vertragsstaat: AT**

1. Verfahren zur Herstellung eines Thiazolderivats der Formel (I)

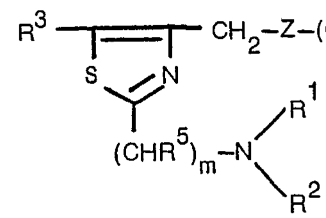

(I)

worin

$R^1$ und $R^2$ unabhängig Wasserstoff, $C_1$—$C_4$-Alkyl, Benzyl oder Benzoyl sind oder zusammen mit dem benachbarten Stickstoffatom einen gesättigten heterocyclischen Ring bilden, der 5 bis 7 Ringatome enthält,

$R^3$ Wasserstoff oder $C_1$—$C_4$-Alkyl ist,

Z für O, S oder $CH_2$ steht,

n für 2 oder 3 steht, falls Z für O oder S steht, und

n für 1, 2 oder 3 steht, falls Z für $CH_2$ steht,

$R^5$ Wasserstoff oder $C_1$—$C_4$-Alkyl ist,

m für 1, 2 oder 3 steht,

Q für 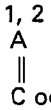 oder 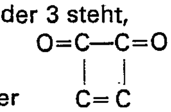 steht,

worin

A für N—CN, N—$NO_2$, CH—$NO_2$, S, O, NH, N—$SO_2$-Aryl, N—$SO_2$—$C_1$—$C_4$-Alkyl, N—CO—$NH_2$, N—CO—$C_1$—$C_4$-Alkyl, N—$CO_2$—$C_1$—$C_4$-Alkyl, CH—$SO_2$-Aryl oder CH—$SO_2$—$C_1$—$C_4$-Alkyl steht, wobei Aryl Phenyl, Halogenphenyl, $C_1$—$C_4$-Alkylphenyl oder $C_1$—$C_4$-Alkoxyphenyl ist, und

B für $NRR^6$ steht, worin R und $R^6$ unabhängig Wasserstoff, $C_1$—$C_5$-Alkyl, $C_3$—$C_6$-Cycloalkylmethyl, Hydroxy-$C_2$—$C_5$-Alkyl, $C_3$—$C_6$-Cycloalkyl, Alkoxyalkyl oder Dialkylaminoalkyl sind, wobei die Gesamtzahl an Kohlenstoffen weniger als 8 beträgt und sich zwischen den Heteroatomen eine Kette aus wenigstens zwei Kohlenstoffatomen befindet, oder eines pharmazeutisch annehmbaren Salzes hiervon, dadurch gekennzeichnet, daß man

(a) ein Amin der Formel

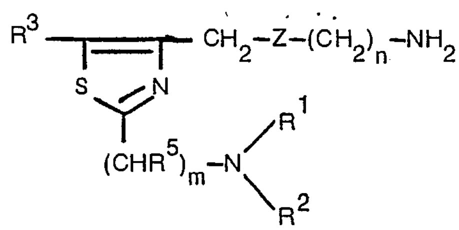

mit

(i) einer Verbindung der Formel $L^1QB$, worin $L^1$ eine Abgangsgruppe ist, oder

(ii) einer Verbindung der Formel $R^6$—N=C=A unter Bildung eines Thiazols der Formel (I), worin Q für —C=A steht und R Wasserstoff ist, umsetzt,

(b) eine Verbindung der Formel

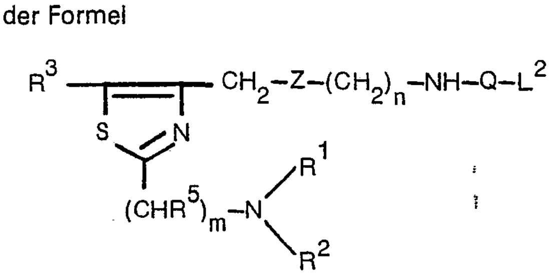

worin $L^2$ eine Abgangsgruppe ist, mit einem Amin der Formel $HNRR^6$ umsetzt,

(c) eine Verbindung der Formel

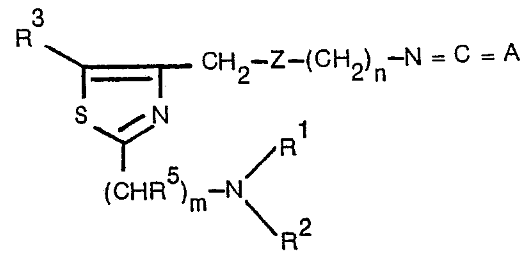

mit einem Amin der Formel $R^6NH_2$ unter Bildung eines Thiazols der Formel (I), worin Q für C=A steht, umsetzt oder

(d) eine Verbindung der Formel

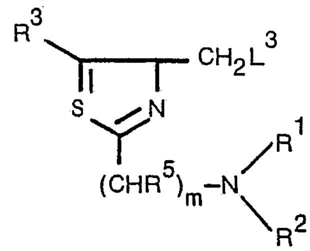

worin $L^3$ eine gute Abgangsgruppe ist, mit einem Thiol der Formel

$$HS(CH_2)_n—NH—CA—NRR^6$$

unter Bildung eines Thiazols der Formel (I), worin Z Schwefel ist und Q für C=A steht, verschmilzt.

2. Verfahren nach Anspruch 1, dadurch gekennzeichnet, daß man ein Thiazolderivat der Formel (I), worin $R^1$ und $R^2$ unabhängig Wasserstoff oder $C_1$—$C_3$-Alkyl bedeuten, nur einer der Substituenten $R^1$ und $R^2$ Benzyl oder Benzoyl ist oder $R^1$ und $R^2$ zusammen mit dem benachbarten Stickstoffatom eine Piperidino-Pyrrolidino- oder Morpholinogruppe darstellt. mit der Maßgabe, daß nur einer der Substituenten $R^1$ und $R^2$ Wasserstoff sein kann, falls Z für $CH_2$ steht, $R^3$ Wasserstoff oder $C_1$—$C_3$-Alkyl ist, $R^4$ für $C_1$—$C_5$-Alkyl steht, $R^5$ Wasserstoff oder Methyl ist, A für N—CN, N—$NO_2$, CH—$NO_2$, S, O, NH, N—$SO_2$-Aryl, N—$SO_2$—$C_1$—$C_3$-Alkyl, N—CO—$NH_2$, N—CO—$C_1$—$C_3$-Alkyl, N—$CO_2$—$C_1$—$C_3$-Alkyl, CH—$SO_2$-Aryl oder CH—$SO_2$—$CH_3$ steht, worin Aryl Phenyl, Halogenphenyl, $C_1$—$C_3$-Alkylphenyl oder $C_1$—$C_3$-Alkoxyphenyl ist und $R^6$ Wasserstoff ist und Q für C=A steht,
oder ein pharmazeutisch annehmbares Säureadditionssalz hiervon herstellt.

3. Verfahren nach Anspruch 1, dadurch gekennzeichnet, daß man N-Methyl-N'-2-([2-(dimethylaminomethyl)-4-thiazolyl]methylthio)ethyl-N"-cyanoguanidin oder ein pharmazeutisch annehmbares Salz hiervon herstellt.

4. Verfahren nach Anspruch 1, dadurch gekennzeichnet, daß man N-Methyl-N'-([2-(dimethylaminomethyl)-4-thiazolyl]methylthio)ethyl-2-nitro-1,1-ethendiamin oder ein pharmazeutisch annehmbares Salz hiervon herstellt.

5. Thiazol der Formel (I) oder ein pharmazeutisch annehmbares Salz hiervon, dadurch gekennzeichnet, daß es nach einem Verfahren nach irgendeinem der Ansprüche 1 bis 4 hergestellt worden ist.